# EUROPEAN PATENT APPLICATION

(11) **EP 1 944 375 A1**
(43) Date of publication of application: **16.07.2008**
(21) Application number: 07290043.4
(22) Date of filing: 11.01.2007
(51) Int. Cl.: C12N 15/82, C12N 9/02, A01H 5/00, A01H 5/10

(54) **FAD2 mutants and high oleic acid plants**

(71) Applicant: Monsanto S.A.S., 69673 Lyon Cedex (FR); Deutsche Saatveredelung AG, 59557 Lippstadt (DE)
(72) Inventor: Despeghel, Jean-Pierre, 45140 Ingre (FR); Wu, Kunsheng, Ballwin MO63021 (US); Busch, Heinrich, 33129 Delbrück (DE)
(74) Representative: pronovem

(57) **Abstract**

The present invention relates to plants, seeds and products derived thereof, in particular to Brassica plants, seeds products derived thereof, that have mutant sequences conferring high oleic acid profile on the seed oil.

More particularly, the invention relates to mutant delta-12 fatty acid desaturase sequences, also referred to herein as FAD2 sequences, in such plants which confer high oleic acid profile on the seed oil

SEQUENCE LISTING

<110>  Monsanto

<120>  FAD2 mutants

<130>  MSTO.10A/EP

<160>  20

<170>  PatentIn version 3.3

<210>  1
<211>  2666
<212>  DNA
<213>  Brassica napus

<400>  1
gagaagagag agagagagag agagagagag agtgagtttg aggaggagct tcttcgtagg      60

gttcatcgtt attaacgtta aatcttcacc ccctacgtca gccagctcaa ggtccctttc     120

ttcttccatt tcttttcatt ctacgttgtt ttcaatctta tgaaactttc tggtctgtgc     180

ttttcttatc gcttttctat tctatctatc atttttgcat ttcagtcgat ttaattctag     240

atctgttaat attaaactat agatctgttc ttgattctct gttttcatgt gtgaaatctg     300

atgctgtatt aatctgatta tattgtctat accgtggaga atatcaaatg ttgcattttc     360

atttgtccga atacaaagtg tttgactttc aatcgttttt aattatatat atatatatat     420

tttttgatgg gttggtggag ttgaaaaatc accatagcag tctcacgtcc tggttttaga     480

aatatcctat tcaaaattat atatttgttt acttgtttta gatctggacc tgagacatat     540

aagtacctat ttgttgaatc tttgggtaaa aacttatgtc tctgggtaaa atttgctggg     600

agatttgacc gattcctatt ggctcttgat tctgtagtta cgtaatacat gaaaaagttt     660

catttggcct atgctcactt catgcttata aacgttttct tgcaaattaa ttggattaga     720

tgttatttca tagattcagt cattcagata caatggagtt gcatgaagaa aataatagaa     780

ttcgtgacag taaaaaagat tgtatttttg tttgtttgtt tatgtttaaa agtctatatg     840

ttgacaatag agttgctctc aactgtttca tttagcttct ttttttgtca agttgcttat     900

tcttagagac attgtgatta tgacttgtct tctttaacgt agtttagtaa taaaagacga     960

aagaaattga tatccacaag aaagagatgt gagctgtagc gtatcaaatc tcgttcattt    1020

actagtagta ttctcaacgc tatcgtttat ttatttttct ttcgttggtt tgccactata    1080

tgccacttct ctcctctttg tcccacgtac tatccatttt ttttgtggta gtccattttc    1140

ttgtaactta taataacgta actctgaatc ttttgtctgt agattaattt gttggtttaa    1200

ttaactttta agtctttgct tttggcttat gcagaaacat gggtgcaggt ggaagaatgc    1260

aagtgtctcc tccctccaag aagtctgaaa ccgacaccat caagcgcgta ccctgcgaga    1320

caccgccctt cactgtcgga gaactcaaga aagcaatccc accgcactgt ttcaaacgct    1380

cgatccctcg ctctttctcc tacctcatct gggacatcat catagcctcc tgcttctact    1440

acgtcgccac cacttacttc cctctcctcc ctcaccctct ctcctacttc gcctggcctc    1500

tctactgggc ctgccaaggg tgcgtcctaa ccggcgtctg ggtcatagcc cacgagtgcg    1560

gccaccacgc cttcagcgac taccagtggt ttgacgacac cgtcggtctc atcttccact    1620

ccttcctcct cgtcccttac ttctcctgga agtacagtca tcgacgccac cattccaaca    1680

ctggctccct cgagagagac gaagtgtttg tccccaagaa gaagtcagac atcaagtggt    1740

acggcaagta cctcaacaac cctttgggac gcaccgtgat gttaacggtt cagttcactc 1800

tcggctggcc gttgtactta gccttcaacg tctcgggaag accttacgac ggcggcttcg 1860

cttgccattt ccaccccaac gcttccatct acaacgaccg cgagcgtctc cagatataca 1920

tctccgacgc tggcatcctc gccgtctgct acggtctctt ccgttacgcc gccgcgcagg 1980

gagtggcctc gatggtctgc ttctacggag tcccgcttct gattgtcaat ggtttcctcg 2040

tgttgatcac ttacttgcag cacacgcatc cttccctgcc tcactacgat tcgtccgagt 2100

gggattggtt gaggggagct ttggctaccg ttgacagaga ctacggaatc ttgaacaagg 2160

tcttccacaa tattaccgac acgcacgtgg cgcatcatct gttctccacg atgccgcatt 2220

atcacgcgat ggaagctacc aaggcgataa agccgatact gggagagtat tatcagttcg 2280

atgggacgcc ggtggttaag gcgatgtgga gggaggcgaa ggagtgtatc tatgtggaac 2340

cggacaggca aggtgagaag aaaggtgtgt tctggtacaa caataagtta tgaggatatg 2400

atgatggtga aagaacaaag aagatattgt cacgaacctt tctcttgctg tctctggtcg 2460

tctttgtttt aagaagctat gttttcgttt caataatctt aactatccat tttgttgtgt 2520

tttctgacat tttggctaaa attatgtgat gttggaagtt agtgtctaaa atgtcttgtg 2580

tctgtattgt tcttcttctc atcgctgtta tgtttgggat cgttgaaatg tgactttcgg 2640

actagtgaac tcttggttct cgaact 2666

<210> 2
<211> 1553
<212> DNA
<213> Brassica napus

<400> 2
gagacagatt cattaccaaa gagatagaga aagagagaga gagagagaga gagagagagt 60

gagtttgagg aggagcttct tcgtagggtt catcgttatt aacgttaaat cttcaccccc 120

tacgtcagcc agctcaagaa acatgggtgc aggtggaaga atgcaagtgt ctcctccctc 180

caagaagtct gaaaccgaca ccatcaagcg cgtaccctgc gagacaccgc ccttcactgt 240

cggagaactc aagaaagcaa tcccaccgca ctgtttcaaa cgctcgatcc ctcgctcttt 300

ctcctacctc atctgggaca tcatcatagc ctcctgcttc tactacgtcg ccaccactta 360

cttccctctc ctccctcacc ctctctccta cttcgcctgg cctctctact gggcctgcca 420

agggtgcgtc ctaaccggcg tctgggtcat agcccacgag tgcggccacc acgccttcag 480

cgactaccag tggcttgacg acaccgtcgg tctcatcttc cactccttcc tcctcgtccc 540

ttacttctcc tggaagtaca gtcatcgacg ccaccattcc aacactggct ccctcgagag 600

agacgaagtg tttgtcccca agaagaagtc agacatcaag tggtacggca agtacctcaa 660

caaccctttg ggacgcaccg tgatgttaac ggttcagttc actctcggct ggccgttgta 720

cttagccttc aacgtctcgg gaagacctta cgacggcggc ttcgcttgcc atttccaccc 780

caacgcttcc atctacaacg accgcgagcg tctccagata tacatctccg acgctggcat 840

cctcgccgtc tgctacggtc tcttccgtta cgccgccgss cagggagtgg cctcgatggt 900

ctgcttctac ggagtcccgc ttctgattgt caatggtttc ctcgtgttga tcacttactt 960

gcagcacacg catccttccc tgcctcacta cgattcgtcc gagtgggatt ggttsagggg 1020

agctttggct accgttgaca gagactacgg aatcttgaac aaggtcttcc acaatattac 1080

cgacacgcac gtggcscatc atcygttctc cacgatgccg cattatcacg cgatggaagc 1140

taccaaggcg ataaagccga tactgggaga gtattatcag ttcgatggga cgccggtggt 1200

taaggcgatg tggagggagg cgaaggagtg tatctatgtg gaaccggaca ggcaaggtga 1260

gaagaaaggt gtgttctggt acaacaataa gttatgagga trraagaaac tgaacctttc 1320

tcttcctatg attgtctttg tttaagaagc tatgtttctg tttcaataat cttaattatc 1380

cattttgttg tgttttctga cattttggct aaaattatgt gatgttggaa gttagtgtct 1440

aaaatgtctt gtgtctgtat tgttcttctt ctcatcgctg ttatgtttgg gatcgttgaa 1500

atgtgacttt cggactagtg aactcttgtt ctcgaactaa aaaaaaaaaa aaa 1553

<210> 3
<211> 1155
<212> DNA
<213> Brassica napus

<220>
<221> CDS
<222> (1)..(1155)

<400> 3
atg ggt gca ggt gga aga atg caa gtg tct cct ccc tcc aag aag tct 48
Met Gly Ala Gly Gly Arg Met Gln Val Ser Pro Pro Ser Lys Lys Ser
1               5                   10                  15

gaa acc gac acc atc aag cgc gta ccc tgc gag aca ccg ccc ttc act 96
Glu Thr Asp Thr Ile Lys Arg Val Pro Cys Glu Thr Pro Pro Phe Thr
            20                  25                  30

gtc gga gaa ctc aag aaa gca atc cca ccg cac tgt ttc aaa cgc tcg 144
Val Gly Glu Leu Lys Lys Ala Ile Pro Pro His Cys Phe Lys Arg Ser
        35                  40                  45

atc cct cgc tct ttc tcc tac ctc atc tgg gac atc atc ata gcc tcc 192
Ile Pro Arg Ser Phe Ser Tyr Leu Ile Trp Asp Ile Ile Ile Ala Ser
    50                  55                  60

tgc ttc tac tac gtc gcc acc act tac ttc cct ctc ctc cct cac cct 240
Cys Phe Tyr Tyr Val Ala Thr Thr Tyr Phe Pro Leu Leu Pro His Pro
65                  70                  75                  80

ctc tcc tac ttc gcc tgg cct ctc tac tgg gcc tgc caa ggg tgc gtc 288
Leu Ser Tyr Phe Ala Trp Pro Leu Tyr Trp Ala Cys Gln Gly Cys Val
                85                  90                  95

cta acc ggc gtc tgg gtc ata gcc cac gag tgc ggc cac cac gcc ttc 336
Leu Thr Gly Val Trp Val Ile Ala His Glu Cys Gly His His Ala Phe
            100                 105                 110

agc gac tac cag tgg ctt gac gac acc gtc ggt ctc atc ttc cac tcc 384
Ser Asp Tyr Gln Trp Leu Asp Asp Thr Val Gly Leu Ile Phe His Ser
        115                 120                 125

ttc ctc ctc gtc cct tac ttc tcc tgg aag tac agt cat cga cgc cac 432
Phe Leu Leu Val Pro Tyr Phe Ser Trp Lys Tyr Ser His Arg Arg His
    130                 135                 140

cat tcc aac act ggc tcc ctc gag aga gac gaa gtg ttt gtc ccc aag 480
His Ser Asn Thr Gly Ser Leu Glu Arg Asp Glu Val Phe Val Pro Lys
145                 150                 155                 160

aag aag tca gac atc aag tgg tac ggc aag tac ctc aac aac cct ttg 528
Lys Lys Ser Asp Ile Lys Trp Tyr Gly Lys Tyr Leu Asn Asn Pro Leu
                165                 170                 175

gga cgc acc gtg atg tta acg gtt cag ttc act ctc ggc tgg ccg ttg 576
Gly Arg Thr Val Met Leu Thr Val Gln Phe Thr Leu Gly Trp Pro Leu
            180                 185                 190

tac tta gcc ttc aac gtc tcg gga aga cct tac gac ggc ggc ttc gct 624

Tyr Leu Ala Phe Asn Val Ser Gly Arg Pro Tyr Asp Gly Gly Phe Ala
        195                 200                 205

tgc cat ttc cac ccc aac gct tcc atc tac aac gac cgc gag cgt ctc       672
Cys His Phe His Pro Asn Ala Ser Ile Tyr Asn Asp Arg Glu Arg Leu
    210                 215                 220

cag ata tac atc tcc gac gct ggc atc ctc gcc gtc tgc tac ggt ctc       720
Gln Ile Tyr Ile Ser Asp Ala Gly Ile Leu Ala Val Cys Tyr Gly Leu
225                 230                 235                 240

ttc cgt tac gcc gcc gcg cag gga gtg gcc tcg atg gtc tgc ttc tac       768
Phe Arg Tyr Ala Ala Ala Gln Gly Val Ala Ser Met Val Cys Phe Tyr
                245                 250                 255

gga gtc ccg ctt ctg att gtc aat ggt ttc ctc gtg ttg atc act tac       816
Gly Val Pro Leu Leu Ile Val Asn Gly Phe Leu Val Leu Ile Thr Tyr
            260                 265                 270

ttg cag cac acg cat cct tcc ctg cct cac tac gat tcg tcc gag tgg       864
Leu Gln His Thr His Pro Ser Leu Pro His Tyr Asp Ser Ser Glu Trp
        275                 280                 285

gat tgg ttg agg gga gct ttg gct acc gtt gac aga gac tac gga atc       912
Asp Trp Leu Arg Gly Ala Leu Ala Thr Val Asp Arg Asp Tyr Gly Ile
    290                 295                 300

ttg aac aag gtc ttc cac aat att acc gac acg cac gtg gcg cat cat       960
Leu Asn Lys Val Phe His Asn Ile Thr Asp Thr His Val Ala His His
305                 310                 315                 320

ctg ttc tcc acg atg ccg cat tat cac gcg atg gaa gct acc aag gcg      1008
Leu Phe Ser Thr Met Pro His Tyr His Ala Met Glu Ala Thr Lys Ala
                325                 330                 335

ata aag ccg ata ctg gga gag tat tat cag ttc gat ggg acg ccg gtg      1056
Ile Lys Pro Ile Leu Gly Glu Tyr Tyr Gln Phe Asp Gly Thr Pro Val
            340                 345                 350

gtt aag gcg atg tgg agg gag gcg aag gag tgt atc tat gtg gaa ccg      1104
Val Lys Ala Met Trp Arg Glu Ala Lys Glu Cys Ile Tyr Val Glu Pro
        355                 360                 365

gac agg caa ggt gag aag aaa ggt gtg ttc tgg tac aac aat aag tta      1152
Asp Arg Gln Gly Glu Lys Lys Gly Val Phe Trp Tyr Asn Asn Lys Leu
    370                 375                 380

tga                                                                  1155

<210>  4
<211>  384
<212>  PRT
<213>  Brassica napus

<400>  4

Met Gly Ala Gly Gly Arg Met Gln Val Ser Pro Pro Ser Lys Lys Ser
1               5                   10                  15

Glu Thr Asp Thr Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Thr Tyr Phe Pro Leu Leu Pro His Pro
65                  70                  75                  80

Leu Ser Tyr Phe Ala Trp Pro Leu Tyr Trp Ala Cys Gln Gly Cys Val
                85                  90                  95

Leu Thr Gly Val Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Asp Gly Gly Phe Ala
        195                 200                 205

Cys His Phe His Pro Asn Ala Ser Ile Tyr Asn Asp Arg Glu Arg Leu
    210                 215                 220

Gln Ile Tyr Ile Ser Asp Ala Gly Ile Leu Ala Val Cys Tyr Gly Leu
225                 230                 235                 240

Phe Arg Tyr Ala Ala Ala Gln Gly Val Ala Ser Met Val Cys Phe Tyr
                245                 250                 255

Gly Val Pro Leu Leu Ile Val Asn Gly Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Ala
                325                 330                 335

Ile Lys Pro Ile Leu Gly Glu Tyr Tyr Gln Phe Asp Gly Thr Pro Val
            340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210> 5
<211> 1561
<212> DNA
<213> Brassica napus

<400> 5
   gagaaccaga gagattcatt accaaagaga tagagagaga gagaaagaga ggagacagag 60
   agagagtttg aggaggagct tcttcgtagg gttcatcgtt attaacgtta aatcttcatc 120
   cccccctacg tcagccagct caagaaacat gggtgcaggt ggaagaatgc aagtgtctcc 180
   tccctccaaa aagtctgaaa ccgacaacat caagcgcgta ccctgcgaga caccgccctt 240
   cactgtcgga gaactcaaga aagcaatccc accgcactgt ttcaaacgct cgatccctcg 300
   ctctttctcc tacctcatct gggacatcat catagcctcc tgcttctact acgtcgccac 360
   cacttacttc cctctcctcc ctcaccctct ctcctacttc gcctggcctc tctactgggc 420
   ctgccagggc tgcgtcctaa ccggcgtctg ggtcatagcc cacgagtgcg gccaccacgc 480
   cttcagcgac taccagtggc tggacgacac cgtcggcctc atcttccact ccttcctcct 540
   cgtcccttac ttctcctgga agtacagtca tcgacgccac cattccaaca ctggctccct 600
   cgagagagac gaagtgtttg tccccaagaa gaagtcagac atcaagtggt acggcaagta 660
   cctcaacaac cctttgggac gcaccgtgat gttaacggtt cagttcactc tcggctggcc 720
   tttgtactta gccttcaacg tctcggggag accttacgac ggcggcttcg cttgccattt 780
   ccaccccaac gctcccatct acaacgaccg tgagcgtctc cagatataca tctccgacgc 840
   tggcatcctc gccgtctgct acggtctcta ccgctacgct gctgtccaag gagttgcctc 900
   gatggtctgc ttctacggag ttcctcttct gattgtcaac gggttcttag ttttgatcac 960
   ttacttgcag cacacgcatc cttccctgcc tcactatgac tcgtctgagt gggattggtt 1020
   gaggggagct ttggccaccg ttgacagaga ctacggaatc ttgaacaagg tcttccacaa 1080
   tatcacggac acgcacgtgg cgcatcacct gttctcgacc atgccgcatt atcatgcgat 1140
   ggaagctacg aaggcgataa agccgatact gggagagtat tatcagttcg atgggacgcc 1200
   ggtggttaag gcgatgtgga gggaggcgaa ggagtgtatc tatgtggaac cggacaggca 1260
   aggtgagaag aaaggtgtgt tctggtacaa caataagtta tgaagcaaag aagaaactga 1320
   acctttctcw tcctatgatt gtctttgttt aagaagctat gtttctgttt caataatctt 1380
   taattatcca ttttgttgtg ttttctgaca ttttggctaa aattatgtga tgttggaagt 1440
   tagtgtctaa aatgtcttgt gtctgtattg ttcttcttct catcgctgtt atgtttggga 1500
   tcgttgaaat gtgactttcg gactagtgaa ctcttgttct cgaactaaaa aaaaaaaaaa 1560
   a 1561

<210> 6
<211> 1155
<212> DNA
<213> Brassica napus

<220>
<221> CDS
<222> (1)..(1155)

<400> 6
atg ggt gca ggt gga aga atg caa gtg tct cct ccc tcc aaa aag tct 48
Met Gly Ala Gly Gly Arg Met Gln Val Ser Pro Pro Ser Lys Lys Ser
1               5                   10                  15

gaa acc gac aac atc aag cgc gta ccc tgc gag aca ccg ccc ttc act 96
Glu Thr Asp Asn Ile Lys Arg Val Pro Cys Glu Thr Pro Pro Phe Thr
            20                  25                  30

gtc gga gaa ctc aag aaa gca atc cca ccg cac tgt ttc aaa cgc tcg 144
Val Gly Glu Leu Lys Lys Ala Ile Pro Pro His Cys Phe Lys Arg Ser
        35                  40                  45

atc cct cgc tct ttc tcc tac ctc atc tgg gac atc atc ata gcc tcc 192
Ile Pro Arg Ser Phe Ser Tyr Leu Ile Trp Asp Ile Ile Ile Ala Ser
    50                  55                  60

tgc ttc tac tac gtc gcc acc act tac ttc cct ctc ctc cct cac cct 240
Cys Phe Tyr Tyr Val Ala Thr Thr Tyr Phe Pro Leu Leu Pro His Pro
65                  70                  75                  80

ctc tcc tac ttc gcc tgg cct ctc tac tgg gcc tgc cag ggc tgc gtc 288
Leu Ser Tyr Phe Ala Trp Pro Leu Tyr Trp Ala Cys Gln Gly Cys Val
                85                  90                  95

cta acc ggc gtc tgg gtc ata gcc cac gag tgc ggc cac cac gcc ttc 336
Leu Thr Gly Val Trp Val Ile Ala His Glu Cys Gly His His Ala Phe
            100                 105                 110

agc gac tac cag tgg ctg gac gac acc gtc ggc ctc atc ttc cac tcc 384
Ser Asp Tyr Gln Trp Leu Asp Asp Thr Val Gly Leu Ile Phe His Ser
        115                 120                 125

ttc ctc ctc gtc cct tac ttc tcc tgg aag tac agt cat cga cgc cac 432
Phe Leu Leu Val Pro Tyr Phe Ser Trp Lys Tyr Ser His Arg Arg His
    130                 135                 140

cat tcc aac act ggc tcc ctc gag aga gac gaa gtg ttt gtc ccc aag 480
His Ser Asn Thr Gly Ser Leu Glu Arg Asp Glu Val Phe Val Pro Lys
145                 150                 155                 160

aag aag tca gac atc aag tgg tac ggc aag tac ctc aac aac cct ttg 528
Lys Lys Ser Asp Ile Lys Trp Tyr Gly Lys Tyr Leu Asn Asn Pro Leu
                165                 170                 175

gga cgc acc gtg atg tta acg gtt cag ttc act ctc ggc tgg cct ttg 576
Gly Arg Thr Val Met Leu Thr Val Gln Phe Thr Leu Gly Trp Pro Leu
            180                 185                 190

tac tta gcc ttc aac gtc tcg ggg aga cct tac gac ggc ggc ttc gct 624
Tyr Leu Ala Phe Asn Val Ser Gly Arg Pro Tyr Asp Gly Gly Phe Ala
        195                 200                 205

tgc cat ttc cac ccc aac gct ccc atc tac aac gac cgt gag cgt ctc 672
Cys His Phe His Pro Asn Ala Pro Ile Tyr Asn Asp Arg Glu Arg Leu
    210                 215                 220

cag ata tac atc tcc gac gct ggc atc ctc gcc gtc tgc tac ggt ctc 720
Gln Ile Tyr Ile Ser Asp Ala Gly Ile Leu Ala Val Cys Tyr Gly Leu
225                 230                 235                 240

tac cgc tac gct gct gtc caa gga gtt gcc tcg atg gtc tgc ttc tac 768
Tyr Arg Tyr Ala Ala Val Gln Gly Val Ala Ser Met Val Cys Phe Tyr
                245                 250                 255

gga gtt cct ctt ctg att gtc aac ggg ttc tta gtt ttg atc act tac 816
Gly Val Pro Leu Leu Ile Val Asn Gly Phe Leu Val Leu Ile Thr Tyr
            260                 265                 270

ttg cag cac acg cat cct tcc ctg cct cac tat gac tcg tct gag tgg 864
Leu Gln His Thr His Pro Ser Leu Pro His Tyr Asp Ser Ser Glu Trp
        275                 280                 285

gat tgg ttg agg gga gct ttg gcc acc gtt gac aga gac tac gga atc 912
Asp Trp Leu Arg Gly Ala Leu Ala Thr Val Asp Arg Asp Tyr Gly Ile
    290                 295                 300

ttg aac aag gtc ttc cac aat atc acg gac acg cac gtg gcg cat cac 960
Leu Asn Lys Val Phe His Asn Ile Thr Asp Thr His Val Ala His His
305                 310                 315                 320

ctg ttc tcg acc atg ccg cat tat cac gcg atg gaa gct acg aag gcg 1008
Leu Phe Ser Thr Met Pro His Tyr His Ala Met Glu Ala Thr Lys Ala
                325                 330                 335

ata aag ccg ata ctg gga gag tat tat cag ttc gat ggg acg ccg gtg 1056
Ile Lys Pro Ile Leu Gly Glu Tyr Tyr Gln Phe Asp Gly Thr Pro Val
            340                 345                 350

gtt aag gcg atg tgg agg gag gcg aag gag tgt atc tat gtg gaa ccg 1104
Val Lys Ala Met Trp Arg Glu Ala Lys Glu Cys Ile Tyr Val Glu Pro
        355                 360                 365

gac agg caa ggt gag aag aaa ggt gtg ttc tgg tac aac aat aag tta 1152
Asp Arg Gln Gly Glu Lys Lys Gly Val Phe Trp Tyr Asn Asn Lys Leu
    370                 375                 380

tga 1155

<210> 7
<211> 384
<212> PRT
<213> Brassica napus

<400> 7
   Met Gly Ala Gly Gly Arg Met Gln Val Ser Pro Pro Ser Lys Lys Ser
   1               5                   10                  15
   Glu Thr Asp Asn Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Val Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Glu Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Arg His
       130                 135                 140
   His Ser Asn Thr Gly Ser Leu Glu Arg Asp Glu Val Phe Val Pro Lys
   145                 150                 155                 160
   Lys Lys Ser Asp Ile Lys Trp Tyr Gly Lys Tyr Leu Asn Asn Pro Leu
                   165                 170                 175
   Gly Arg Thr Val Met Leu Thr Val Gln Phe Thr Leu Gly Trp Pro Leu
               180                 185                 190
   Tyr Leu Ala Phe Asn Val Ser Gly Arg Pro Tyr Asp Gly Gly Phe Ala
   195                 200                 205
   Cys His Phe His Pro Asn Ala Pro Ile Tyr Asn Asp Arg Glu Arg Leu
       210                 215                 220
   Gln Ile Tyr Ile Ser Asp Ala Gly Ile Leu Ala Val Cys Tyr Gly Leu
   225                 230                 235                 240
   Tyr Arg Tyr Ala Ala Val Gln Gly Val Ala Ser Met Val Cys Phe Tyr
                   245                 250                 255
   Gly Val Pro Leu Leu Ile Val Asn Gly Phe Leu Val Leu Ile Thr Tyr
               260                 265                 270
   Leu Gln His Thr His Pro Ser Leu Pro His Tyr Asp Ser Ser Glu Trp
           275                 280                 285
   Asp Trp Leu Arg Gly Ala Leu Ala Thr Val Asp Arg Asp Tyr Gly Ile
       290                 295                 300
   Leu Asn Lys Val Phe His Asn Ile Thr Asp Thr His Val Ala His His
   305                 310                 315                 320
   Leu Phe Ser Thr Met Pro His Tyr His Ala Met Glu Ala Thr Lys Ala
                   325                 330                 335
   Ile Lys Pro Ile Leu Gly Glu Tyr Tyr Gln Phe Asp Gly Thr Pro Val
               340                 345                 350
   Val Lys Ala Met Trp Arg Glu Ala Lys Glu Cys Ile Tyr Val Glu Pro
           355                 360                 365
   Asp Arg Gln Gly Glu Lys Lys Gly Val Phe Trp Tyr Asn Asn Lys Leu
       370                 375                 380

<210>  8
<211>  2614
<212>  DNA
<213>  Brassica napus

<400>  8
agagagagaa gagaggagac agagagagag tttgaggagg agcttcttcg tagggttcat      60

cgttattaac gttaaatctt catccccccc tacgtcagcc agctcaaggt ccctttcttc     120

ttccatttct tctcattttt acgttgtttt caatcttggt ctgttctttt cttatcgctt     180

ttctgttcta tctatcattt ttgcatttca gtcgatttat ttctagatct gttaatattt     240

attgcattaa actatagatc tggtcttgat tctctgtttt catgtgtgaa atcttgatgc     300

tgtcttaacc attaatctga ttatattgtc tataccgtgg agaatatgaa atgttgcatt     360

ttcatttgtc cgaatacaaa ctgtttgact ttcaatcgtt tttaaaatta tatatatatt     420

tttgatgggt tggtggagtt gaaaaatcac catagcagtc tcacgtcctg gtcttagaaa     480

tatccttcct attcaaagtt atatatattt gtttactttt gttttagatc tggacctgag     540

acatgtaagt acatatttgt tgaatctttg ggtaaaaaac ttatgtctct gggtaaaatt     600

tgctgagaga tttgaccgat tcctattggc tctggattct gtagttacct aatacatgaa     660

aaagtttcat ttggcctatg ctcacttcat gcttataaac tttttcttgc aaattaattg     720

gattagatgc tccttcatag attcagatgc aatagatttg catgaagaaa ataataggat 780
tcatgatagt aaaaaagatt gtatttttgt ttgtttgttt atgtttaaaa gtctatatgt 840
tgacaataga gttgctatca actgtttcat ttaggtttat gtttttgtca agttgcttat 900
tctaagagac attgtgatta tgacttgtct tctctaacgt agtttagtaa taaaagacga 960
aagaaattga tatccacaag aaagagatgt aagctgtaac gtatcaaatc tcattaataa 1020
ctagtagtat tctcaacgct atcgtttatt tctttctttg gtttgccact atatgccgct 1080
tctctgctct tttatcccac gtactatcca ttttttttgt ggtagtccat ttttttgaaa 1140
ctttaataac gtaacactga atattaattt gttggtttaa ttaactttga gtctttgctt 1200
ttggtttatg cagaaacatg ggtgcaggtg gaagaatgca agtgtctcct ccctccaaaa 1260
agtctgaaac cgacaacatc aagcgcgtac cctgcgagac accgcccttc actgtcggag 1320
aactcaagaa agcaatccca ccgcactgtt tcaaacgctc gatccctcgc tctttctcct 1380
acctcatctg ggacatcatc atagcctcct gcttctacta cgtcgccacc acttacttcc 1440
ctctcctccc tcaccctctc tcctacttcg cctggcctct ctactgggcc tgccagggct 1500
gcgtcctaac cggcgtctgg gtcatagccc acgagtgcgg ccaccacgcc ttcagcgact 1560
accagtggct ggacgacacc gtcggcctca tcttccactc cttcctcctc gtcccttact 1620
tctcctggaa gtacagtcat cgacgccacc attccaacac tggctccctc gagagagacg 1680
aagtgtttgt ccccaagaag aagtcagaca tcaagtggta cggcaagtac ctcaacaacc 1740
ctttgggacg caccgtgatg ttaacggttc agttcactct cggctggcct ttgtacttag 1800
ccttcaacgt ctcggggaga ccttacgacg gcggcttcgc ttgccatttc caccccaacg 1860
ctcccatcta caacgaccgt gagcgtctcc agatatacat ctccgacgct ggcatcctcg 1920
ccgtctgcta cggtctctac cgctacgctg ctgtccaagg agttgcctcg atggtctgct 1980
tctacggagt tcctcttctg attgtcaacg ggttcttagt tttgatcact tacttgcagc 2040
acacgcatcc ttccctgcct cactatgact cgtctgagtg ggattggttg aggggagctt 2100
tggccaccgt tgacagagac tacggaatct tgaacaaggt cttccacaat atcacggaca 2160
cgcacgtggc gcatcacctg ttctcgacca tgccgcatta tcacgcgatg gaagctacga 2220
aggcgataaa gccgatactg ggagagtatt atcagttcga tgggacgccg gtggttaagg 2280
cgatgtggag ggaggcgaag gagtgtatct atgtggaacc ggacaggcaa ggtgagaaga 2340
aaggtgtgtt ctggtacaac aataagttat gaagcaaaga agaaactgaa cctttctcat 2400
ctatgattgt ctttgtttta agaagctatg tttctgtttc aataatcttt aattatccat 2460
tttgttgtgt tttctgacat tttggctaaa attatgtgat gttggaagtt agtgtctaaa 2520
atgtcttgtg tctgtattgt tcttcttctc atcgctgtta tgtttgggat cgttgaaatg 2580
tgactttcgg actagtgaat cttgttctcg aact 2614

<210> 9
<211> 1561
<212> DNA
<213> Brassica napus

<400> 9
gagaaccaga gagattcatt accaaagaga tagagagaga gagaaagaga ggagacagag 60
agagagtttg aggaggagct tcttcgtagg gttcatcgtt attaacgtta aatcttcatc 120

cccccctacg tcagccagct caagaaacat gggtgcaggt ggaagaatgc aagtgtctcc 180
tccctccaaa aagtctgaaa ccgacaacat caagcgcgta ccctgcgaga caccgccctt 240
cactgtcgga gaactcaaga aagcaatccc accgcactgt ttcaaacgct cgatccctcg 300
ctctttctcc tacctcatct gggacatcat catagcctcc tgcttctact acgtcgccac 360
cacttacttc cctctcctcc ctcaccctct ctcctacttc gcctggcctc tctactgggc 420
ctgccagggc tgcgtcctaa ccggcgtctg ggtcatagcc cacgagtgcg gccaccacgc 480
cttcagcgac taccagtggc tggacgacac cgtcggcctc atcttccact ccttcctcct 540
cgtcccttac ttctcctgga agtacagtca tcgacgccac cattccaaca ctggctccct 600
cgagagagac gaagtgtttg tccccaagaa gaagtcagac atcaagtggt acggcaagta 660
cctcaacaac cctttgggac gcaccgtgat gttaacggtt cagttcactc tcggctggcc 720
tttgtactta gccttcaacg tctcggggag accttacgac ggcggcttcg cttgccattt 780
ccaccccaac gctcccatct acaacgaccg tgagcgtctc cagatataca tctccgacgc 840
tggcatcctc gccgtctgct acggtctcta ccgctacgct gctgtccaag gagttgcctc 900
gatggtctgc ttctacggag ttcctcttct gattgtcaac gggttcttag ttttgatcac 960
ttacttgcag cacacgcatc cttccctgcc tcactatgac tcgtctgagt gggattggtt 1020
gaggggagct ttggccaccg ttgacagaga ctacggaatc ttgaacaagg tcttccacaa 1080
tatcacggac acgcacgtgg cgcatcacct gttctcgacc atgccgcatt atcatgcgat 1140
ggaagctacg aaggcgataa agccgatact gggagagtat tatcagttcg atgggacgcc 1200
ggtggttaag gcgatgtgga gggaggcgaa ggagtgtatc tatgtggaac cggacaggca 1260
aggtgagaag aaaggtgtgt tctggtacaa caataagtta tgaagcaaag aagaaactga 1320
acctttctcw tcctatgatt gtctttgttt aagaagctat gtttctgttt caataatctt 1380
taattatcca ttttgttgtg ttttctgaca ttttggctaa aattatgtga tgttggaagt 1440
tagtgtctaa aatgtcttgt gtctgtattg ttcttcttct catcgctgtt atgtttggga 1500
tcgttgaaat gtgactttcg gactagtgaa ctcttgttct cgaactaaaa aaaaaaaaaa 1560
a 1561

<210> 10
<211> 1155
<212> DNA
<213> Brassica napus

<220>
<221> CDS
<222> (1)..(1155)

<400> 10
atg ggt gca ggt gga aga atg caa gtg tct cct ccc tcc aaa aag tct 48
Met Gly Ala Gly Gly Arg Met Gln Val Ser Pro Pro Ser Lys Lys Ser
1               5                   10                  15

gaa acc gac aac atc aag cgc gta ccc tgc gag aca ccg ccc ttc act 96
Glu Thr Asp Asn Ile Lys Arg Val Pro Cys Glu Thr Pro Pro Phe Thr
            20                  25                  30

gtc gga gaa ctc aag aaa gca atc cca ccg cac tgt ttc aaa cgc tcg 144
Val Gly Glu Leu Lys Lys Ala Ile Pro Pro His Cys Phe Lys Arg Ser
        35                  40                  45

atc cct cgc tct ttc tcc tac ctc atc tgg gac atc atc ata gcc tcc 192
Ile Pro Arg Ser Phe Ser Tyr Leu Ile Trp Asp Ile Ile Ile Ala Ser

    50                  55                  60

tgc ttc tac tac gtc gcc acc act tac ttc cct ctc ctc cct cac cct 240
Cys Phe Tyr Tyr Val Ala Thr Thr Tyr Phe Pro Leu Leu Pro His Pro
65                  70                  75                  80

ctc tcc tac ttc gcc tgg cct ctc tac tgg gcc tgc cag ggc tgc gtc 288
Leu Ser Tyr Phe Ala Trp Pro Leu Tyr Trp Ala Cys Gln Gly Cys Val
                85                  90                  95

cta acc ggc gtc tgg gtc ata gcc cac gag tgc ggc cac cac gcc ttc 336
Leu Thr Gly Val Trp Val Ile Ala His Glu Cys Gly His His Ala Phe
            100                 105                 110

agc gac tac cag tgg ctg gac gac acc gtc ggc ctc atc ttc cac tcc 384
Ser Asp Tyr Gln Trp Leu Asp Asp Thr Val Gly Leu Ile Phe His Ser
        115                 120                 125

ttc ctc ctc gtc cct tac ttc tcc tgg aag tac agt cat cga cgc cac 432
Phe Leu Leu Val Pro Tyr Phe Ser Trp Lys Tyr Ser His Arg Arg His
    130                 135                 140

cat tcc aac act ggc tcc ctc gag aga gac gaa gtg ttt gtc ccc aag 480
His Ser Asn Thr Gly Ser Leu Glu Arg Asp Glu Val Phe Val Pro Lys
145                 150                 155                 160

aag aag tca gac atc aag tgg tac ggc aag tac ctc aac aac cct ttg 528
Lys Lys Ser Asp Ile Lys Trp Tyr Gly Lys Tyr Leu Asn Asn Pro Leu
                165                 170                 175

gga cgc acc gtg atg tta acg gtt cag ttc act ctc ggc tgg cct ttg 576
Gly Arg Thr Val Met Leu Thr Val Gln Phe Thr Leu Gly Trp Pro Leu
            180                 185                 190

tac tta gcc ttc aac gtc tcg ggg aga cct tac gac ggc ggc ttc gct 624
Tyr Leu Ala Phe Asn Val Ser Gly Arg Pro Tyr Asp Gly Gly Phe Ala
        195                 200                 205

tgc cat ttc cac ccc aac gct ccc atc tac aac gac cgt gag cgt ctc 672
Cys His Phe His Pro Asn Ala Pro Ile Tyr Asn Asp Arg Glu Arg Leu
    210                 215                 220

cag ata tac atc tcc gac gct ggc atc ctc gcc gtc tgc tac ggt ctc 720
Gln Ile Tyr Ile Ser Asp Ala Gly Ile Leu Ala Val Cys Tyr Gly Leu
225                 230                 235                 240

tac cgc tac gct gct gtc caa gga gtt gcc tcg atg gtc tgc ttc tac 768
Tyr Arg Tyr Ala Ala Val Gln Gly Val Ala Ser Met Val Cys Phe Tyr
                245                 250                 255

gga gtt cct ctt ctg att gtc aac ggg ttc tta gtt ttg atc act tac 816
Gly Val Pro Leu Leu Ile Val Asn Gly Phe Leu Val Leu Ile Thr Tyr
            260                 265                 270

ttg cag cac acg cat cct tcc ctg cct cac tat gac tcg tct gag tgg 864
Leu Gln His Thr His Pro Ser Leu Pro His Tyr Asp Ser Ser Glu Trp
        275                 280                 285

gat tgg ttg agg gga gct ttg gcc acc gtt gac aga gac tac gga atc 912
Asp Trp Leu Arg Gly Ala Leu Ala Thr Val Asp Arg Asp Tyr Gly Ile
    290                 295                 300

ttg aac aag gtc ttc cac aat atc acg gac acg cac gtg gcg cat cac 960
Leu Asn Lys Val Phe His Asn Ile Thr Asp Thr His Val Ala His His
305                 310                 315                 320

ctg ttc tcg acc atg ccg cat tat cac gcg atg gaa gct acg aag gcg 1008
Leu Phe Ser Thr Met Pro His Tyr His Ala Met Glu Ala Thr Lys Ala
                325                 330                 335

ata aag ccg ata ctg gga gag tat tat cag ttc gat ggg acg ccg gtg 1056
Ile Lys Pro Ile Leu Gly Glu Tyr Tyr Gln Phe Asp Gly Thr Pro Val
            340                 345                 350

gtt aag gcg atg tgg agg gag gcg aag gag tgt atc tat gtg gaa ccg 1104
Val Lys Ala Met Trp Arg Glu Ala Lys Glu Cys Ile Tyr Val Glu Pro

355                 360                 365

gac agg caa ggt gag aag aaa ggt gtg ttc tgg tac aac aat aag tta      1152
Asp Arg Gln Gly Glu Lys Lys Gly Val Phe Trp Tyr Asn Asn Lys Leu
    370                 375                 380

tga                                                                  1155

<210>  11
<211>  384
<212>  PRT
<213>  Brassica napus

<400>  11

Met Gly Ala Gly Gly Arg Met Gln Val Ser Pro Pro Ser Lys Lys Ser
1               5                   10                  15

Glu Thr Asp Asn Ile Lys Arg Val Pro Cys Glu Thr Pro Pro Phe Thr
            20                  25                  30

Val Gly Glu Leu Lys Lys Ala Ile Pro Pro His Cys Phe Lys Arg Ser
        35                  40                  45

Ile Pro Arg Ser Phe Ser Tyr Leu Ile Trp Asp Ile Ile Ile Ala Ser
    50                  55                  60

Cys Phe Tyr Tyr Val Ala Thr Thr Tyr Phe Pro Leu Leu Pro His Pro
65                  70                  75                  80

Leu Ser Tyr Phe Ala Trp Pro Leu Tyr Trp Ala Cys Gln Gly Cys Val
                85                  90                  95

Leu Thr Gly Val Trp Val Ile Ala His Glu Cys Gly His His Ala Phe
            100                 105                 110

Ser Asp Tyr Gln Trp Leu Asp Asp Thr Val Gly Leu Ile Phe His Ser
        115                 120                 125

Phe Leu Leu Val Pro Tyr Phe Ser Trp Lys Tyr Ser His Arg Arg His
    130                 135                 140

His Ser Asn Thr Gly Ser Leu Glu Arg Asp Glu Val Phe Val Pro Lys
145                 150                 155                 160

Lys Lys Ser Asp Ile Lys Trp Tyr Gly Lys Tyr Leu Asn Asn Pro Leu
                165                 170                 175

Gly Arg Thr Val Met Leu Thr Val Gln Phe Thr Leu Gly Trp Pro Leu
            180                 185                 190

Tyr Leu Ala Phe Asn Val Ser Gly Arg Pro Tyr Asp Gly Gly Phe Ala
        195                 200                 205

Cys His Phe His Pro Asn Ala Pro Ile Tyr Asn Asp Arg Glu Arg Leu
    210                 215                 220

Gln Ile Tyr Ile Ser Asp Ala Gly Ile Leu Ala Val Cys Tyr Gly Leu
225                 230                 235                 240

Tyr Arg Tyr Ala Ala Val Gln Gly Val Ala Ser Met Val Cys Phe Tyr
                245                 250                 255

Gly Val Pro Leu Leu Ile Val Asn Gly Phe Leu Val Leu Ile Thr Tyr
            260                 265                 270

Leu Gln His Thr His Pro Ser Leu Pro His Tyr Asp Ser Ser Glu Trp
        275                 280                 285

Asp Trp Leu Arg Gly Ala Leu Ala Thr Val Asp Arg Asp Tyr Gly Ile
    290                 295                 300

Leu Asn Lys Val Phe His Asn Ile Thr Asp Thr His Val Ala His His
305                 310                 315                 320

Leu Phe Ser Thr Met Pro His Tyr His Ala Met Glu Ala Thr Lys Ala
                325                 330                 335

Ile Lys Pro Ile Leu Gly Glu Tyr Tyr Gln Phe Asp Gly Thr Pro Val
            340                 345                 350

Val Lys Ala Met Trp Arg Glu Ala Lys Glu Cys Ile Tyr Val Glu Pro
        355                 360                 365

Asp Arg Gln Gly Glu Lys Lys Gly Val Phe Trp Tyr Asn Asn Lys Leu
    370                 375                 380

<210>  12
<211>  2666
<212>  DNA
<213>  Brassica napus

<400>  12
gagaagagag agagagagag agagagagag agtgagtttg aggaggagct tcttcgtagg      60

gttcatcgtt attaacgtta aatcttcacc ccctacgtca gccagctcaa ggtccctttc     120

ttcttccatt tcttttcatt ctacgttgtt ttcaatctta tgaaactttc tggtctgtgc     180

ttttcttatc gcttttctat tctatctatc atttttgcat ttcagtcgat ttaattctag     240

atctgttaat attaaactat agatctgttc ttgattctct gttttcatgt gtgaaatctg     300

atgctgtatt aatctgatta tattgtctat accgtggaga atatcaaatg ttgcattttc     360

atttgtccga atacaaagtg tttgactttc aatcgttttt aattatatat atatatatat     420

tttttgatgg gttggtggag ttgaaaaatc accatagcag tctcacgtcc tggttttaga     480

aatatcctat tcaaaattat atatttgttt acttgtttta gatctggacc tgagacatat     540

aagtacctat ttgttgaatc tttgggtaaa aacttatgtc tctgggtaaa atttgctggg     600

agatttgacc gattcctatt ggctcttgat tctgtagtta cgtaatacat gaaaaagttt     660

catttggcct atgctcactt catgcttata aacgttttct tgcaaattaa ttggattaga     720

tgttatttca tagattcagt cattcagata caatggagtt gcatgaagaa aataatagaa     780

ttcgtgacag taaaaaagat tgtatttttg tttgtttgtt tatgtttaaa agtctatatg     840

ttgacaatag agttgctctc aactgtttca tttagcttct ttttttgtca agttgcttat     900

tcttagagac attgtgatta tgacttgtct tctttaacgt agtttagtaa taaaagacga     960

aagaaattga tatccacaag aaagagatgt gagctgtagc gtatcaaatc tcgttcattt    1020

actagtagta ttctcaacgc tatcgtttat ttatttttct ttcgttggtt tgccactata 1080
tgccacttct ctcctctttg tcccacgtac tatccatttt ttttgtggta gtccattttc 1140
ttgtaactta taataacgta actctgaatc ttttgtctgt agattaattt gttggtttaa 1200
ttaactttta agtctttgct tttggcttat gcagaaacat gggtgcaggt ggaagaatgc 1260
aagtgtctcc tccctccaag aagtctgaaa ccgacaccat caagcgcgta ccctgcgaga 1320
caccgccctt cactgtcgga gaactcaaga aagcaatccc accgcactgt ttcaaacgct 1380
cgatccctcg ctctttctcc tacctcatct gggacatcat catagcctcc tgcttctact 1440
acgtcgccac cacttacttc cctctcctcc ctcaccctct ctcctacttc gcctggcctc 1500
tctactgggc ctgccaaggg tgcgtcctaa ccggcgtctg ggtcatagcc cacgagtgcg 1560
gccaccacgc cttcagcgac taccagtggt ttgacgacac cgtcggtctc atcttccact 1620
ccttcctcct cgtcccttac ttctcctgga agtacagtca tcgacgccac cattccaaca 1680
ctggctccct cgagagagac gaagtgtttg tccccaagaa gaagtcagac atcaagtggt 1740
acggcaagta cctcaacaac cctttgggac gcaccgtgat gttaacggtt cagttcactc 1800
tcggctggcc gttgtactta gccttcaacg tctcgggaag accttacgac ggcggcttcg 1860
cttgccattt ccaccccaac gctcccatct acaacgaccg cgagcgtctc cagatataca 1920
tctccgacgc tggcatcctc gccgtctgct acggtctctt ccgttacgcc gccgcgcagg 1980
gagtggcctc gatggtctgc ttctacggag tcccgcttct gattgtcaat ggtttcctcg 2040
tgttgatcac ttacttgcag cacacgcatc cttccctgcc tcactacgat tcgtccgagt 2100
gggattggtt gaggggagct ttggctaccg ttgacagaga ctacggaatc ttgaacaagg 2160
tcttccacaa tattaccgac acgcacgtgg cgcatcatct gttctccacg atgccgcatt 2220
atcacgcgat ggaagctacc aaggcgataa agccgatact gggagagtat tatcagttcg 2280
atgggacgcc ggtggttaag gcgatgtgga gggaggcgaa ggagtgtatc tatgtggaac 2340
cggacaggca aggtgagaag aaaggtgtgt tctggtacaa caataagtta tgaggatatg 2400
atgatggtga aagaacaaag aagatattgt cacgaacctt tctcttgctg tctctggtcg 2460
tctttgtttt aagaagctat gttttcgttt caataatctt aactatccat tttgttgtgt 2520
tttctgacat tttggctaaa attatgtgat gttggaagtt agtgtctaaa atgtcttgtg 2580
tctgtattgt tcttcttctc atcgctgtta tgtttgggat cgttgaaatg tgactttcgg 2640
actagtgaac tcttggttct cgaact 2666

<210> 13
<211> 1553
<212> DNA
<213> Brassica napus

<400> 13
gagacagatt cattaccaaa gagatagaga aagagagaga gagagagaga gagagagagt 60
gagtttgagg aggagcttct tcgtagggtt catcgttatt aacgttaaat cttcaccccc 120
tacgtcagcc agctcaagaa acatgggtgc aggtggaaga atgcaagtgt ctcctccctc 180
caagaagtct gaaaccgaca ccatcaagcg cgtaccctgc gagacaccgc ccttcactgt 240
cggagaactc aagaaagcaa tcccaccgca ctgtttcaaa cgctcgatcc ctcgctcttt 300
ctcctacctc atctgggaca tcatcatagc ctcctgcttc tactacgtcg ccaccactta 360
cttccctctc ctccctcacc ctctctccta cttcgcctgg cctctctact gggcctgcca 420

agggtgcgtc ctaaccggcg tctgggtcat agcccacgag tgcggccacc acgccttcag 480
cgactaccag tggcttgacg acaccgtcgg tctcatcttc cactccttcc tcctcgtccc 540
ttacttctcc tggaagtaca gtcatcgacg ccaccattcc aacactggct ccctcgagag 600
agacgaagtg tttgtcccca agaagaagtc agacatcaag tggtacggca agtacctcaa 660
caaccctttg ggacgcaccg tgatgttaac ggttcagttc actctcggct ggccgttgta 720
cttagccttc aacgtctcgg gaagacctta cgacggcggc ttcgcttgcc atttccaccc 780
caacgctccc atctacaacg accgcgagcg tctccagata tacatctccg acgctggcat 840
cctcgccgtc tgctacggtc tcttccgtta cgccgccgss cagggagtgg cctcgatggt 900
ctgcttctac ggagtcccgc ttctgattgt caatggtttc ctcgtgttga tcacttactt 960
gcagcacacg catccttccc tgcctcacta cgattcgtcc gagtgggatt ggttsagggg 1020
agctttggct accgttgaca gagactacgg aatcttgaac aaggtcttcc acaatattac 1080
cgacacgcac gtggcscatc atcygttctc cacgatgccg cattatcacg cgatggaagc 1140
taccaaggcg ataaagccga tactgggaga gtattatcag ttcgatggga cgccggtggt 1200
taaggcgatg tggagggagg cgaaggagtg tatctatgtg gaaccggaca ggcaaggtga 1260
gaagaaaggt gtgttctggt acaacaataa gttatgagga trraagaaac tgaacctttc 1320
tcttcctatg attgtctttg tttaagaagc tatgtttctg tttcaataat cttaattatc 1380
cattttgttg tgttttctga cattttggct aaaattatgt gatgttggaa gttagtgtct 1440
aaaatgtctt gtgtctgtat tgttcttctt ctcatcgctg ttatgtttgg gatcgttgaa 1500
atgtgacttt cggactagtg aactcttgtt ctcgaactaa aaaaaaaaaa aaa 1553

<210> 14
<211> 1155
<212> DNA
<213> Brassica napus

<220>
<221> CDS
<222> (1)..(1155)

<400> 14
atg ggt gca ggt gga aga atg caa gtg tct cct ccc tcc aag aag tct 48
Met Gly Ala Gly Gly Arg Met Gln Val Ser Pro Pro Ser Lys Lys Ser
1               5                   10                  15

gaa acc gac acc atc aag cgc gta ccc tgc gag aca ccg ccc ttc act 96
Glu Thr Asp Thr Ile Lys Arg Val Pro Cys Glu Thr Pro Pro Phe Thr
            20                  25                  30

gtc gga gaa ctc aag aaa gca atc cca ccg cac tgt ttc aaa cgc tcg 144
Val Gly Glu Leu Lys Lys Ala Ile Pro Pro His Cys Phe Lys Arg Ser
        35                  40                  45

atc cct cgc tct ttc tcc tac ctc atc tgg gac atc atc ata gcc tcc 192
Ile Pro Arg Ser Phe Ser Tyr Leu Ile Trp Asp Ile Ile Ile Ala Ser
    50                  55                  60

tgc ttc tac tac gtc gcc acc act tac ttc cct ctc ctc cct cac cct 240
Cys Phe Tyr Tyr Val Ala Thr Thr Tyr Phe Pro Leu Leu Pro His Pro
65                  70                  75                  80

ctc tcc tac ttc gcc tgg cct ctc tac tgg gcc tgc caa ggg tgc gtc 288
Leu Ser Tyr Phe Ala Trp Pro Leu Tyr Trp Ala Cys Gln Gly Cys Val
                85                  90                  95

cta acc ggc gtc tgg gtc ata gcc cac gag tgc ggc cac cac gcc ttc 336

Leu Thr Gly Val Trp Val Ile Ala His Glu Cys Gly His His Ala Phe
            100                 105                 110

agc gac tac cag tgg ctt gac gac acc gtc ggt ctc atc ttc cac tcc 384
Ser Asp Tyr Gln Trp Leu Asp Asp Thr Val Gly Leu Ile Phe His Ser
        115                 120                 125

ttc ctc ctc gtc cct tac ttc tcc tgg aag tac agt cat cga cgc cac 432
Phe Leu Leu Val Pro Tyr Phe Ser Trp Lys Tyr Ser His Arg Arg His
    130                 135                 140

cat tcc aac act ggc tcc ctc gag aga gac gaa gtg ttt gtc ccc aag 480
His Ser Asn Thr Gly Ser Leu Glu Arg Asp Glu Val Phe Val Pro Lys
145                 150                 155                 160

aag aag tca gac atc aag tgg tac ggc aag tac ctc aac aac cct ttg 528
Lys Lys Ser Asp Ile Lys Trp Tyr Gly Lys Tyr Leu Asn Asn Pro Leu
                165                 170                 175

gga cgc acc gtg atg tta acg gtt cag ttc act ctc ggc tgg ccg ttg 576
Gly Arg Thr Val Met Leu Thr Val Gln Phe Thr Leu Gly Trp Pro Leu
            180                 185                 190

tac tta gcc ttc aac gtc tcg gga aga cct tac gac ggc ggc ttc gct 624
Tyr Leu Ala Phe Asn Val Ser Gly Arg Pro Tyr Asp Gly Gly Phe Ala
        195                 200                 205

tgc cat ttc cac ccc aac gct ccc atc tac aac gac cgc gag cgt ctc 672
Cys His Phe His Pro Asn Ala Pro Ile Tyr Asn Asp Arg Glu Arg Leu
    210                 215                 220

cag ata tac atc tcc gac gct ggc atc ctc gcc gtc tgc tac ggt ctc 720
Gln Ile Tyr Ile Ser Asp Ala Gly Ile Leu Ala Val Cys Tyr Gly Leu
225                 230                 235                 240

ttc cgt tac gcc gcc gcg cag gga gtg gcc tcg atg gtc tgc ttc tac 768
Phe Arg Tyr Ala Ala Ala Gln Gly Val Ala Ser Met Val Cys Phe Tyr
                245                 250                 255

gga gtc ccg ctt ctg att gtc aat ggt ttc ctc gtg ttg atc act tac 816
Gly Val Pro Leu Leu Ile Val Asn Gly Phe Leu Val Leu Ile Thr Tyr
            260                 265                 270

ttg cag cac acg cat cct tcc ctg cct cac tac gat tcg tcc gag tgg 864
Leu Gln His Thr His Pro Ser Leu Pro His Tyr Asp Ser Ser Glu Trp
        275                 280                 285

gat tgg ttg agg gga gct ttg gct acc gtt gac aga gac tac gga atc 912
Asp Trp Leu Arg Gly Ala Leu Ala Thr Val Asp Arg Asp Tyr Gly Ile
    290                 295                 300

ttg aac aag gtc ttc cac aat att acc gac acg cac gtg gcg cat cat 960
Leu Asn Lys Val Phe His Asn Ile Thr Asp Thr His Val Ala His His
305                 310                 315                 320

ctg ttc tcc acg atg ccg cat tat cac gcg atg gaa gct acc aag gcg 1008
Leu Phe Ser Thr Met Pro His Tyr His Ala Met Glu Ala Thr Lys Ala
                325                 330                 335

ata aag ccg ata ctg gga gag tat tat cag ttc gat ggg acg ccg gtg 1056
Ile Lys Pro Ile Leu Gly Glu Tyr Tyr Gln Phe Asp Gly Thr Pro Val
            340                 345                 350

gtt aag gcg atg tgg agg gag gcg aag gag tgt atc tat gtg gaa ccg 1104
Val Lys Ala Met Trp Arg Glu Ala Lys Glu Cys Ile Tyr Val Glu Pro
        355                 360                 365

gac agg caa ggt gag aag aaa ggt gtg ttc tgg tac aac aat aag tta 1152
Asp Arg Gln Gly Glu Lys Lys Gly Val Phe Trp Tyr Asn Asn Lys Leu
    370                 375                 380

tga 1155

<210> 15
<211> 384

<212>  PRT
<213>  Brassica napus

<400>  15
   Met Gly Ala Gly Gly Arg Met Gln Val Ser Pro Pro Ser Lys Lys Ser
   1               5                   10                  15
   Glu Thr Asp Thr Ile Lys Arg Val Pro Cys Glu Thr Pro Pro Phe Thr
               20                  25                  30
   Val Gly Glu Leu Lys Lys Ala Ile Pro Pro His Cys Phe Lys Arg Ser
           35                  40                  45
   Ile Pro Arg Ser Phe Ser Tyr Leu Ile Trp Asp Ile Ile Ile Ala Ser
       50                  55                  60
   Cys Phe Tyr Tyr Val Ala Thr Thr Tyr Phe Pro Leu Leu Pro His Pro
   65                  70                  75                  80
   Leu Ser Tyr Phe Ala Trp Pro Leu Tyr Trp Ala Cys Gln Gly Cys Val
                   85                  90                  95
   Leu Thr Gly Val Trp Val Ile Ala His Glu Cys Gly His His Ala Phe
               100                 105                 110
   Ser Asp Tyr Gln Trp Leu Asp Asp Thr Val Gly Leu Ile Phe His Ser
           115                 120                 125
   Phe Leu Leu Val Pro Tyr Phe Ser Trp Lys Tyr Ser His Arg Arg His
       130                 135                 140
   His Ser Asn Thr Gly Ser Leu Glu Arg Asp Glu Val Phe Val Pro Lys
   145                 150                 155                 160
   Lys Lys Ser Asp Ile Lys Trp Tyr Gly Lys Tyr Leu Asn Asn Pro Leu
                   165                 170                 175
   Gly Arg Thr Val Met Leu Thr Val Gln Phe Thr Leu Gly Trp Pro Leu
               180                 185                 190
   Tyr Leu Ala Phe Asn Val Ser Gly Arg Pro Tyr Asp Gly Gly Phe Ala
           195                 200                 205
   Cys His Phe His Pro Asn Ala Pro Ile Tyr Asn Asp Arg Glu Arg Leu
       210                 215                 220
   Gln Ile Tyr Ile Ser Asp Ala Gly Ile Leu Ala Val Cys Tyr Gly Leu
   225                 230                 235                 240
   Phe Arg Tyr Ala Ala Ala Gln Gly Val Ala Ser Met Val Cys Phe Tyr
                   245                 250                 255
   Gly Val Pro Leu Leu Ile Val Asn Gly Phe Leu Val Leu Ile Thr Tyr
               260                 265                 270
   Leu Gln His Thr His Pro Ser Leu Pro His Tyr Asp Ser Ser Glu Trp
           275                 280                 285
   Asp Trp Leu Arg Gly Ala Leu Ala Thr Val Asp Arg Asp Tyr Gly Ile
       290                 295                 300
   Leu Asn Lys Val Phe His Asn Ile Thr Asp Thr His Val Ala His His
   305                 310                 315                 320
   Leu Phe Ser Thr Met Pro His Tyr His Ala Met Glu Ala Thr Lys Ala
                   325                 330                 335
   Ile Lys Pro Ile Leu Gly Glu Tyr Tyr Gln Phe Asp Gly Thr Pro Val
               340                 345                 350
   Val Lys Ala Met Trp Arg Glu Ala Lys Glu Cys Ile Tyr Val Glu Pro
           355                 360                 365
   Asp Arg Gln Gly Glu Lys Lys Gly Val Phe Trp Tyr Asn Asn Lys Leu
       370                 375                 380

<210>  16
<211>  18
<212>  DNA
<213>  Brassica napus

<400>  16
gccttcaacg tctcggga                                                    18

<210>  17
<211>  24
<212>  DNA
<213>  Brassica napus

<400>  17
gagatgtata tctggagacg ctcg                                             24

<210>  18
<211>  15
<212>  DNA
<213>  Brassica napus

<400>  18
ccaacgctcc catct                                                       15

<210>  19
<211>  15
<212>  DNA
<213>  Brassica napus

<400>  19
ccaacgcttc catct                                                       15

<210>  20
<211>  2614
<212>  DNA
<213>  Brassica napus

<220>
<221>  misc_feature
<222>  (406)..(407)
<223>  n is a, c, g, or t

<220>
<221>  misc_feature
<222>  (418)..(419)
<223>  n is a, c, g, or t

<220>
<221>  misc_feature
<222>  (500)..(501)
<223>  n is a, c, g, or t

<220>
<221>  misc_feature
<222>  (574)..(574)
<223>  n is a, c, g, or t

<220>
<221>  misc_feature
<222>  (579)..(579)
<223>  n is a, c, g, or t

<220>
<221>  misc_feature
<222>  (644)..(652)
<223>  n is a, c, g, or t

<220>
<221>  misc_feature
<222>  (672)..(672)
<223>  n is a, c, g, or t

<220>
<221>  misc_feature
<222>  (791)..(791)
<223>  n is a, c, g, or t

<220>
<221>  misc_feature
<222>  (1090)..(1090)
<223>  n is a, c, g, or t

<220>
<221>  misc_feature
<222>  (2416)..(2416)
<223>  n is a, c, g, or t

<220>
<221>  misc_feature
<222>  (2448)..(2448)
<223>  n is a, c, g, or t

<400>  20
agagagagaa gagaggagac agagagagag tttgaggagg agcttcttcg tagggttcat      60

cgttattaac gttaaatctt catccccccc tacgtcagcc agctcaaggt ccctttcttc     120

ttccatttct tctcattttt acgttgtttt caatcttggt ctgttctttt cttatcgctt     180

ttctgttcta tctatcattt ttgcatttca gtcgatttaa ttctagatct gttaatattt     240

attgcattaa actatagatc tggtctcgat tctctgtttt catgtgtgaa atcttgatgc     300

tgtctttacc attaatctgc ttatattgta tataccgtgg agaatatgaa atgttgcatt     360

ttcatttgtc cgaatacaaa ctgtttgact tccaatcgtt tttaanntta tatatatnnt     420

tttgatgggt tggtggagtt gaaaaatcac catagcagtc tcacgtcctg gttttagaaa     480

tatccttcct attcaaagtn ntatatattt gtttactttt gttttagatc tggacctgag     540

acatgtaagt acatatttgt tgaatctttg ggtnaaaant ttatgtctct gggtaaaatt     600

tgctgagaga tttgaccgat tcctattggc tctggattct gtannnnnnn nntacatgaa     660

aaagtttcat tnggcctatg ctcacgtcat gcttacaaac ttttctttgc aaattaattc     720

gattagatgc tccttcatag attcagatgc aatagatttg catgaagaaa ataataggat     780

tcatgatagt naaaaagatt gtacattttt ttgtttgttt atgtttaaaa gtctatatgt     840

tgacaataga gttgctatca actgtttcat ttaggttttt gtttttctca agttgcttat     900

tcttagagac attgtgatta tgacttgtcg tctttaacgt agtttagtaa taaaagacga 960
aagaaattga tatccacaag aaagagatgt gagctgtagc gtatcaaatc tcattaataa 1020
ctagtagtat tctcaacgct atcgtttatt tctttctttg gtttgccact atatgccgct 1080
tctctgctcn tttatcccac gtactatcca ttttttttgt ggtagtccat ttttttgaaa 1140
ctttaataac gtaacactga atattaattt gttggtttaa ttaactttga gtctttgctt 1200
ttggtttatg cagaaacatg ggtgcaggtg gaagaatgca agtgtctcct ccctccaaaa 1260
agtctgaaac cgacaacatc aagcgcgtac cctgcgagac accgcccttc actgtcggag 1320
aactcaagaa agcaatccca ccgcactgtt tcaaacgctc gatccctcgc tctttctcct 1380
acctcatctg ggacatcatc atagcctcct gcttctacta cgtcgccacc acttacttcc 1440
ctctcctccc tcaccctctc tcctacttcg cctggcctct ctactgggcc tgccagggct 1500
gcgtcctaac cggcgtctgg gtcatagccc acgagtgcgg ccaccacgcc ttcagcgact 1560
accagtggct ggacgacacc gtcggcctca tcttccactc cttcctcctc gtcccttact 1620
tctcctggaa gtacagtcat cgacgccacc attccaacac tggctccctc gagagagacg 1680
aagtgtttgt ccccaagaag aagtcagaca tcaagtggta cggcaagtac ctcaacaacc 1740
ctttgggacg caccgtgatg ttaacggttc agttcactct cggctggcct ttgtacttag 1800
ccttcaacgt ctcggggaga ccttacgacg gcggcttcgc ttgccatttc caccccaacg 1860
ctcccatcta caacgaccgt gagcgtctcc agatatacat ctccgacgct ggcatcctcg 1920
ccgtctgcta cggtctctac cgctacgctg ctgtccaagg agttgcctcg atggtctgct 1980
tctacggagt tcctcttctg attgtcaacg ggttcttagt tttgatcact tacttgcagc 2040
acacgcatcc ttccctgcct cactatgact cgtctgagtg ggattggttg aggggagctt 2100
tggccaccgt tgacagagac tacggaatct tgaacaaggt cttccacaat atcacggaca 2160
cgcacgtggc gcatcacctg ttctcgacca tgccgcatta tcatgcgatg gaagctacga 2220
aggcgataaa gccgatactg ggagagtatt atcagttcga tgggacgccg gtggttaagg 2280
cgatgtggag ggaggcgaag gagtgtatct atgtggaacc ggacaggcaa ggtgagaaga 2340
aaggtgtgtt ctggtacaac aataagttat gaagcaaaga agaaactgaa cctttctctt 2400
ctatgattgt ctttgnttta agaagctatg tttctgtttc aataatcntt aattatccat 2460
tttgttgtgt tttctgacat tttggctaaa attatgtgat gttggaagtt agtgtctaaa 2520
atgtcttgtg tctgtattgt tcttcttctc atcgctgtta tgtttgggat cgttgaaatg 2580
tgactttcgg actagtgaat cttgttctcg aact 2614

## Description

### Field of the invention

The present invention relates to plants, seeds and products derived thereof, in particular to Brassica plants, seeds products derived thereof, that have mutant sequences conferring high oleic acid profile to the seed oil.

More particularly, the invention relates to mutant delta-12 fatty acid desaturase sequences, also referred to herein as FAD2 sequences, in such plants which confer high oleic acid profile on the seed oil.

### Background

Delta-12 fatty acid desaturase (also known as oleic desaturase or oleate desaturase) is involved in the enzymatic conversion of oleic acid to linoleic acid.

Varieties with high level of oleic acid (possibly combined with low level of linolenic acid) are sought for many different applications (food applications, health applications, biodiesel applications and many others).

Mutant seeds providing an oil exhibiting a high oleic acid content (oleic acid content higher that 70 wt.% based upon the total weight of fatty acids present in the oil) previously reported in the literature had very poor agronomic value and/or bad root characteristics, and/or very low yield capacity and/Or bad germination capacity and/or an oleic acid content stability problem across environments.

There is still a need for material having stable, high oleic acid content (possibly combined with stable low linolenic acid content) across locations and across years, with also good germination, good agronomic performances and with normal oilseed rape morphology. In particular, the plants should have no fasciation and should have normal root development.

### Summary of the invention

The present invention relates to a nucleic acid molecule comprising (or consisting of) a nucleic acid sequence encoding a delta-12 oleate desaturase (FAD2) protein, said FAD2 protein having an amino acid substitution at position 216 relative to a wild-type FAD2 protein.

Preferably, said FAD2 protein is a Brassica FAD2 protein, more particularly a *Brassica napus* FAD2 protein.

Preferably, said substituted amino acid at position 216 is a serine (replacing the proline at position 216 of a wild-type FAD2 protein).

A preferred nucleic acid molecule of the invention comprises (or consists of) a nucleic acid of SEQ ID NO 1, 2, or 3, its complementary form or its RNA form.

A nucleic acid molecule of the invention can comprise or consist of a nucleotide sequence having at least 80%, preferably at least 85%, more preferably at least 90% and even more preferably at least 95%, 96%, 97%, 98% or 99% identity with SEQ ID NO 1, 2 or 3, or with the complementary form or RNA form thereof, encoding a FAD2 protein having an amino acid substitution at position 216 relative to a wild-type FAD2 protein.

More particularly, said wild-type FAD2 protein comprises (or consists of) an amino acid sequence of SEQ ID NO 11 or 15.

Also object of the present invention is a fragment of at least 10, 15, 20, 25, 30, 40, 50, 100 or more nucleotides of a nucleic acid molecule according to the invention, said fragment comprising the mutated codon corresponding to said amino acid substitution at position 216.

Said fragments can be used as primers, probes and/or selectable markers.

Any of the nucleic acid molecules of the invention can be used in a method of marker assisted selection of plants, preferably of Brassica species, more preferably of *Brassica napus* varieties, also object of the present invention.

Another object of the present invention is an assay kit which can comprise a first container containing any of the nucleic acid molecules of the invention.

Another object of the present invention is a FAD2 protein having an amino acid substitution at position 216, or corresponding to position 216, relative to a wild-type FAD2 protein such as the wild-type FAD2 protein represented by the amino acid sequence of SEQ ID NO 11 or 15.

A preferred FAD2 protein of the invention comprises (or consists of) an amino acid sequence of SEQ ID NO 4.

Another object of the present invention is a vector comprising a nucleic acid molecule encoding a mutant FAD2 protein according to the invention.

Another object of the present invention is a host cell comprising a vector of the invention and/or a nucleic acid sequence encoding a mutant FAD2 protein according to the invention.

Another object of the present invention is a plant stably transformed with a vector of the invention.

A plant to be transformed can be selected from the group consisting of oil producing crops, more particularly, from sunflowers, soybeans, cottons, corns and/or rapeseeds.

Another object of the present invention is a plant or a plant part or a seed containing a nucleotide sequence encoding a FAD-2 protein having an amino acid substitution at or corresponding to position 216 relative to a wild-type FAD-2 protein.

More particularly, a plant or a plant part or a seed according to the invention contains (or expresses) a FAD-2 protein having an amino acid substitution at or corresponding to position 216 relative to a wild-type FAD-2 protein.

Preferably, said substituted amino acid at or corresponding to position 216 is a serine (replacing a proline at position 216 of a wild-type FAD2 protein).

A plant or a plant part or a seed according to the invention can be obtained by a mutagenesis treatment, more particularly by an EMS treatment.

Progenies derived from said plant or plant part or seed are also objects of the invention.

Another object of the present invention is a vegetable oil obtained from seeds of the invention, said oil comprising more than (about) 80%, 81%, 82%, 83%, 84%, 85%, 86% or 87% of oleic acid based upon the total weight of the fatty acids present in the rapeseed oil.

Preferably, said oil further comprises less than (about) 4%, 3,5%, 3%, 2%, 1% or 0,5% of linolenic acid.

The invention also relates to food or feed products containing and/or prepared with a plant, a plant part, a seed and/or a vegetable oil according to the invention.

A method of enhancing the oleic acid content in a plant can comprise the step of transforming a plant with a vector of the invention.

Alternatively, a method of producing high oleic plant lines can comprise:
(a) crossing a first plant of the invention with a second plant,
(b) obtaining seeds from the cross of step (a),
(c) growing fertile plants from such seeds,
(d) obtaining progeny seeds from the plants of step(c), and
(e) identifying those seeds among the progeny that have high oleic acid content.

Alternatively, a method of producing high oleic plant lines can comprise:
(a) inducing mutagenesis in at least some cells from a plant, more particularly of a Brassica plant, and preferably of a *Brassica napus* variety that has a oleic acid content of less than 70%;
(b) regenerating plants from at least one of said mutagenized cells;
(c) selecting regenerated plants which have any of the nucleic acid sequences of the invention and/or which expresses a FAD2 protein according to the invention; and
(d) deriving further generations of plants from said regenerated plants.

### Brief description of the figure

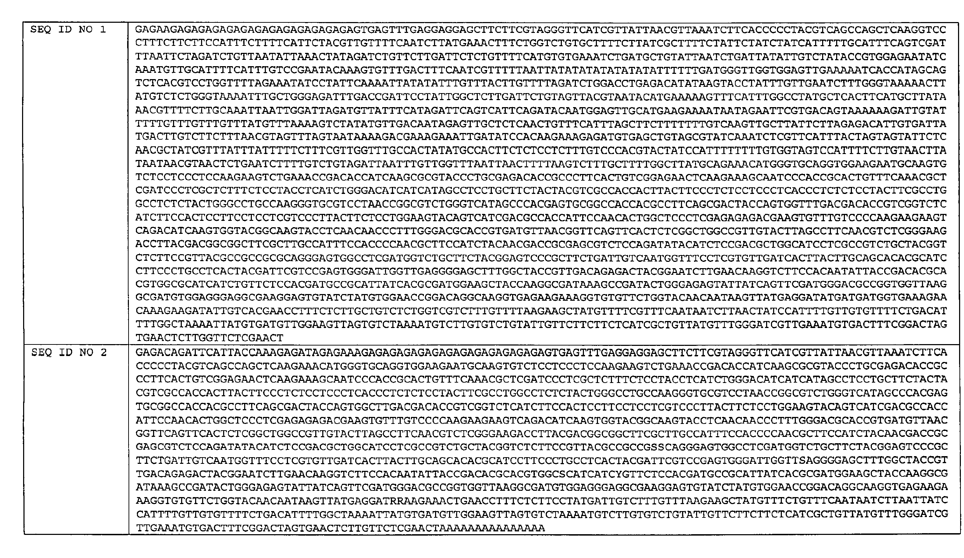

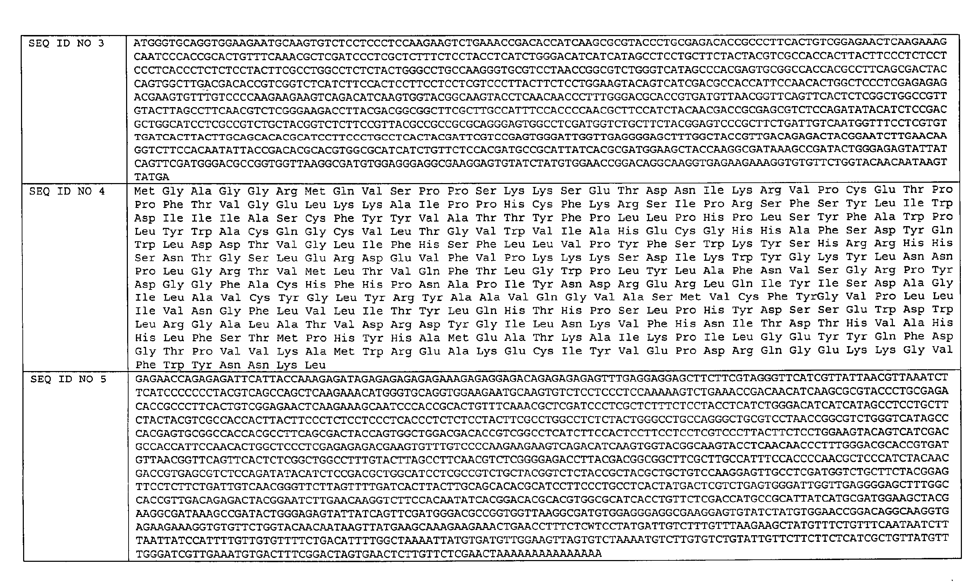

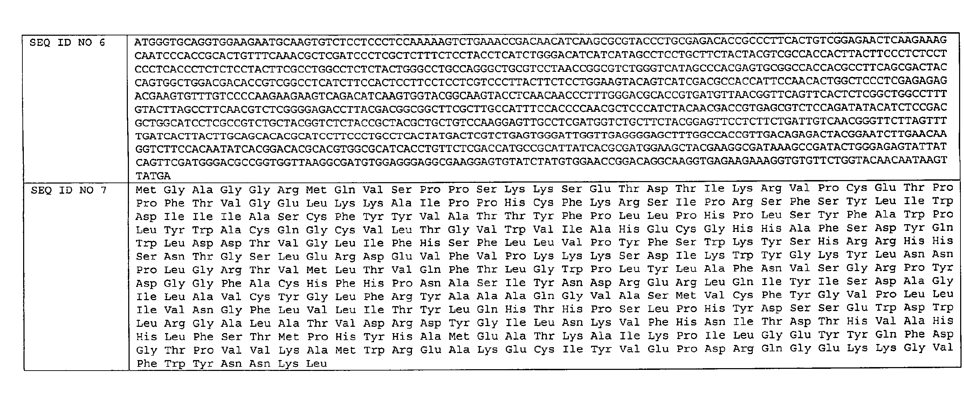

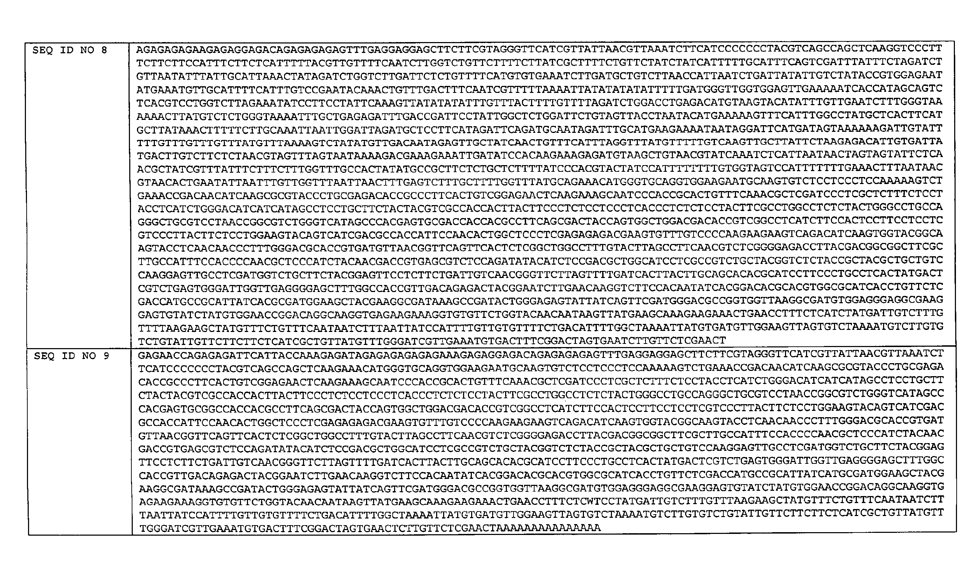

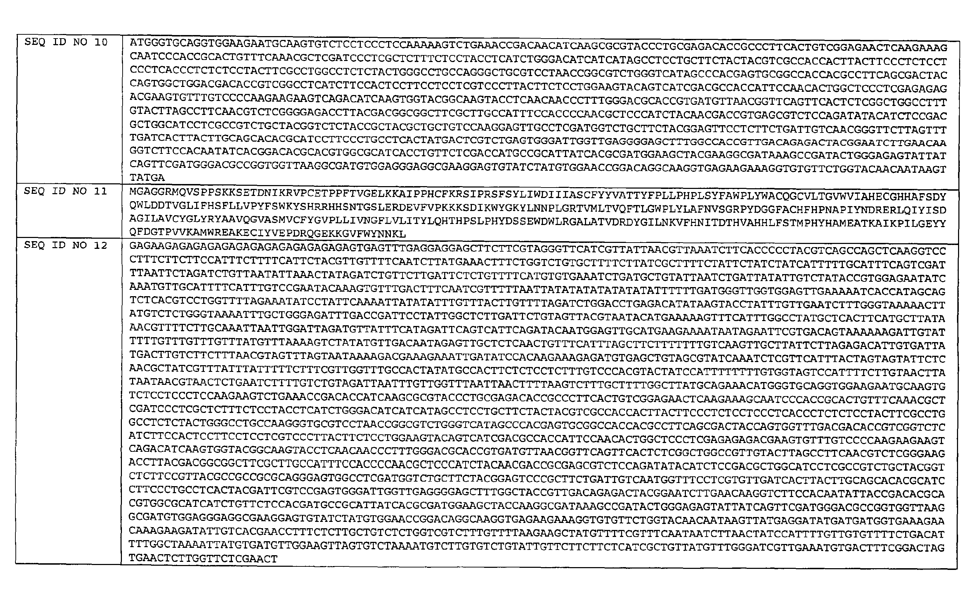

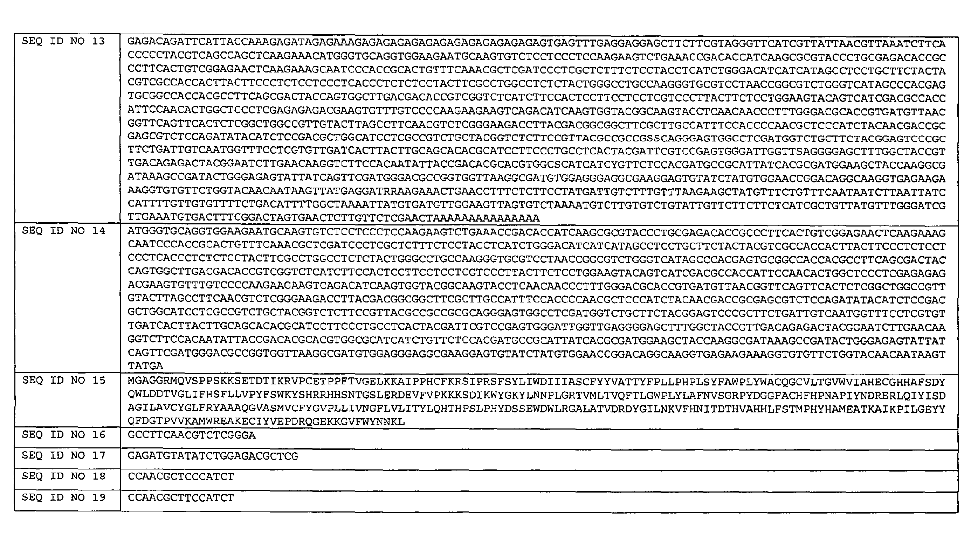

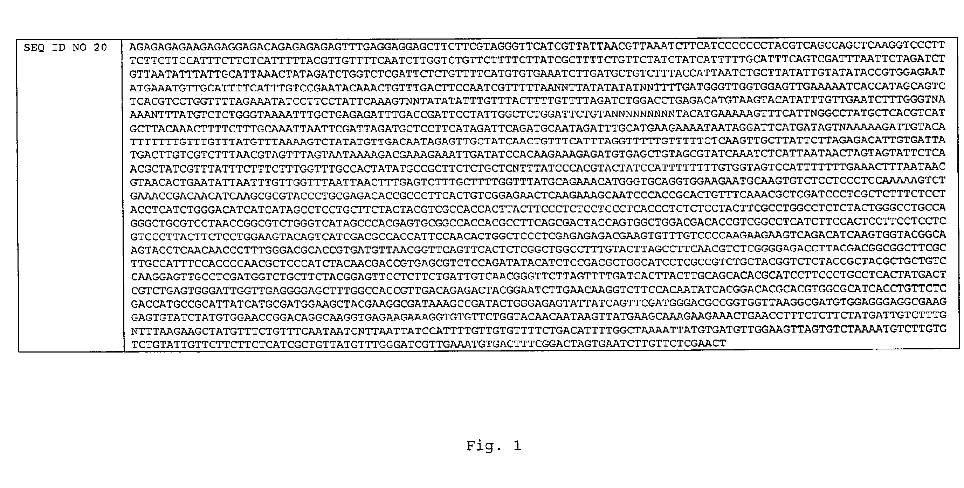

Figure 1 corresponds to the list of sequences of the present invention.

### Detailed description of the invention

The present invention relates to plants, more particularly to Brassica plants, preferably to *Brassica napus* varieties, which have been created for providing an oil having a oleic acid content higher than 80 wt.%, based upon the total weight of fatty acids present in the oil.

More particularly, a plant of the invention has at least one mutated FAD2 gene of the invention.

Preferably, said mutated FAD2 gene confers very high oleic acid content (i.e. a oleic acid content higher than 80 wt.%, based upon the total weight of fatty acids present in the oil) in seeds of said plants and in oil extracted from said seeds.

The present invention relates also to any part or any product of said plant bearing said at least one mutated FAD2 gene.

In the context of the present invention, a part or product of a plant is meant to encompass a leaf, cotyledon, stem, petiole, stalk, seed or any other tissue or fragment of tissue of said plant.

The present invention relates also to any progeny of said plant bearing said at least one mutated FAD2 gene of the invention.

In the context of the present invention, the term "progeny" refers to direct and indirect descendants, offspring and derivatives of a plant or plants of the invention and includes the first, second, third and/or subsequent generations, which may be produced by self crossing, crossing with plants with the same or different genotypes, and may be modified by range of suitable genetic engineering techniques.

The present invention also relates to said mutated FAD2 genes that confer high oleic acid content in seeds when present in a plant.

In particular, the invention relates to novel isolated nucleic acid molecules that encode novel variant forms of FAD2 protein having a substituted amino acid at position 216 (or corresponding to position 216) relative to a wild-type FAD2 protein, such as the wild-type FAD2 protein represented by SEQ ID NO 11 or more particularly by SEQ ID NO 15.

An isolated nucleic acid molecule of the invention contains said at least one mutation, resulting in a substitution, preferably a substitution of serine for proline, at (or corresponding to) position 216 relative to a wild-type FAD2 protein, such as the wild-type FAD2 protein represented by SEQ ID NO 11, or more particularly by SEQ ID NO 15.

Said mutation(s) alter(s) the functionality of the resulting FAD2 gene product, whereby the level of oleic acid is modified, preferably increased, in plant expressing the mutant sequence(s), compared to the corresponding level in plant expressing the wild-type sequence(s).

In the framework of the present invention, except if otherwise specified, the term "at position 216" is to be understood as designating the amino acid position 216 in a wild-type FAD2 protein represented by SEQ ID NO 11 or more particularly by SEQ ID NO 15, but also as referring to the amino acid corresponding to said position in a wild-type FAD2 protein that would have a different amino acid sequence due to deletions or additional amino acids in the polypeptide.

The term "corresponding to position" as used herein means that a position is not only determined by the number of the preceding amino acids. The position of a given amino acid in accordance with the present invention may vary due to deletions or additional amino acids in the polypeptide. Thus, under a "corresponding position" in accordance with the present invention it is to be understood that the amino acid(s) referred to may differ in the indicated number but still has (have) similar neighbouring amino acids in the linear sequence.

In one aspect, a nucleic acid molecule of the invention encodes a FAD2 protein having a substitution of a non-ionisable polar amino acid for a proline at position 216 relative to a wild type FAD2 protein, such as the wild type FAD2 protein represented by the amino acid sequence of SEQ ID NO 11 or more particularly of SEQ ID NO 15.

Said non-ionisable polar amino acid can be threonine, asparagine, glutamine, cysteine, tyrosine or serine. Preferably, said non-ionisable polar amino acid is serine.

A nucleic acid molecule of the invention can be derived (be generated, or be designed) from a nucleic acid molecule having a nucleic acid sequence of SEQ ID NO 5, 6, 8, 9, 10, 12, 13, 14 or 20.

Indeed, a nucleic acid molecule of the invention can comprise (or consist of) a nucleic acid sequence of SEQ ID NO 5, 6, 8, 9, 10, 12, 13, 14 or 20 wherein the codon encoding the amino acid at position 216 (or corresponding to position 216) of said wild-type FAD2 protein is mutated to encode an amino acid different from proline, or preferably to encode a non-ionisable polar amino acid (e.g. a threonine, asparagine, glutamine, cysteine, or a tyrosine), or more preferably to encode a serine.

Preferably, a nucleic acid molecule of the invention encodes a FAD2 protein having a substitution of a serine for a proline at position 216 (or corresponding to position 216) relative to a wild type FAD2 protein, such as the wild type FAD2 protein represented by the amino acid sequence of SEQ ID NO 11 or 15.

A preferred nucleic acid molecule of the invention comprises (or consists of) a nucleic acid sequence of SEQ ID NO 1, 2 or 3.

A nucleic acid molecule of the invention can be derived (generated, or designed) from a nucleic acid molecule having a nucleic acid sequence of SEQ ID NO 1, 2 or 3.

Indeed, a nucleic acid molecule of the invention can comprise (or consist of) a nucleic acid sequence of SEQ ID NO 1, 2 or 3, wherein the codon TCC encoding said serine at position 216 is mutated to encode an amino acid different from proline, or preferably to encode a non-ionisable polar amino acid (e.g. a threonine, asparagine, glutamine, cysteine, or a tyrosine), or more preferably to encode a serine.

Also object of the present invention is a fragment of at least 10, 15, 20, 25, 30, 40, 50, 100 or more nucleotides of a nucleic acid molecule according to the invention, said fragment comprising the mutated codon corresponding to said amino acid substitution at position 216.

In another aspect, a nucleic acid molecule of the invention can encode a FAD2 protein having a deletion at position 216 relative to a wild type FAD2 protein, such as a wild type FAD2 protein represented by the amino acid sequence of SEQ ID NO 11 or 15.

More particularly, a nucleic acid molecule of the invention can encode a FAD2 protein having a proline deleted at position 216 relative to a wild type FAD2 protein represented by the amino acid sequence of SEQ ID NO 11 or 15.

A nucleic acid molecule of the invention can be derived (generated, or designed) from a nucleic acid molecule having a nucleic acid sequence of SEQ ID NO 1, 2, 3, 5, 6, 8, 9, 10, 12, 13, 14 or 20.

Indeed, a nucleic acid molecule of the invention can comprise (or consist of) a nucleic acid sequence of SEQ ID NO 1, 2, 3, 5, 6, 8, 9, 10, 12, 13, 14 or 20 wherein the codon encoding the amino acid at position 216 (or corresponding to position 216) is deleted.

Also object of the present invention is a fragment of at least 10, 15, 20, 25, 30, 40, 50, 100 or more nucleotides of a nucleic acid molecule according to the invention, said fragment comprising the deletion of said codon encoding said amino acid at position 216 (or corresponding to said position 216).

It will be appreciated by the skilled person that the nucleic acid sequences of SEQ ID NO 1-3, 5, 6, 8-10, 12-14, and 20 are not the only sequences that can be used to provide a FAD2 protein of the invention. Also contemplated are any nucleic acid molecules having different sequences but which, because of the degeneracy of the genetic code, encode a FAD2 protein comprising a substitution of an amino acid at position 216 (or corresponding to position 216) relative to the wild-type amino acid sequence, such as the wild-type FAD2 protein represented by SEQ ID NO 11 or 15.

In particular, a nucleic acid molecule of the invention can comprise (or consist of) a nucleotide sequence having at least 80%, preferably at least 85%, more preferably at least 90% and even more preferably at least 95%, 96%, 97%, 98% or 99% identity with any of SEQ ID NO 1-3, 5, 6, 8-10, 12-14, 20, or with the complementary form or RNA form thereof, encoding a FAD2 protein having an amino acid substitution at position 216 (or corresponding to position 216) relative to a wild-type FAD2 protein, such as the wild-type FAD2 protein represented by SEQ ID NO 11 or 15.

A nucleic acid molecule of the invention can be derived from *Brassica napus* varieties, such as SPE04300-075, OSE270, OSPE487, or PyL 616 varieties.

More particularly, a nucleic acid molecule of the invention has a mutation at position 1884 (also referred to as SNP1884) of the acid nucleic sequence of SEQ ID NO 1, which causes a change in genetic codon from CCC to TCC, resulting in a substitution of a serine for a proline at position 216 relative to the wild-type amino acid sequence represented by SEQ ID NO 15.

An isolated nucleic acid molecule of the invention containing said SNP1884 mutation, resulting in a substitution of a serine for a proline at position 216, alters the functionality of the resulting FAD2 gene product, whereby the level of oleic acid is increased in plant expressing the mutant sequence, compared to the corresponding level in plant expressing the wild-type sequence.

In the framework of the invention, the term "SNP1884" refers to the single nucleotide polymorphism corresponding to said mutation at position 1884 of the nucleic acid of SEQ ID NO 1, and can refer also to the corresponding mutation in any nucleic acid molecule encoding a FAD2 protein of the invention, i.e. a FAD2 protein having a substituted amino acid at position 216 (or corresponding to position 216), and in particular having a substitution of a serine for a proline at position 216, relative to the wild-type FAD2 protein, such as the wild-type FAD2 protein represented by SEQ ID NO 11 or 15.

Any fragment of a nucleic acid molecule of the invention of at least 10, 15, 20, 25, 50, 100 or more nucleotides comprising said SNP1884 is contemplated. Examples of fragments are nucleic acid represented by SEQ ID NO 16 to 19.

Such fragments can be used as primers, as probes and/or as markers.

The nucleic acid fragments of the invention can be used as markers in plant genetic mapping and plant breeding programs.

Such markers may include restriction fragment length polymorphism (RFLP), random amplification polymorphism detection (RAPD), polymerase chain reaction (PCR) or self-sustained sequence replication (3SR) markers, for example.

Marker-assisted breeding techniques may be used to identify and follow a plant according to the invention or its progeny, also object of the invention, during the breeding process.

Marker-assisted breeding techniques may be used in addition to, or as an alternative to, other sorts of identification techniques.

An example of marker-assisted breeding is the use of PCR primers that specifically amplify a nucleic acid molecule of the invention.

The invention thereby provides methods for segregation and selection analysis of genetic crosses involving plants having nucleic acid sequences of the invention.

A method of the invention may for example involve determining the presence in a genome of particular FAD2 alleles containing at least one mutation resulting in a substitution (preferably a substitution of serine for proline) at (or corresponding to) position 216 relative to a wild type FAD2 protein, such as the wild type FAD2 protein represented by SEQ ID NO 11 or preferably 15.

Such a determination may for example be achieved with a range of techniques, such as PCR amplification, DNA fingerprinting, RNA fingerprinting, gel blotting and RFLP analysis, nuclease protection assays, sequencing of the relevant nucleic acid fragment, the generation of antibodies (monoclonal or polyclonal), or alternative methods adapted to distinguish the protein produced by the relevant alleles from other variant forms of that protein or from the wild-type.

More particularly, such fragments can be used in method of marker assisted selection for high oleic traits in plants, preferably in Brassica species, more particularly in *Brassica napus* varieties.

Another aspect of the present invention is related to a recombinant nucleotide sequence comprising, operably linked to a nucleotide sequence according to the invention, one or more adjacent regulatory sequence(s). Said adjacent regulatory sequence(s) is/are preferably originating from homologous organisms.

However said adjacent regulatory sequences may also be originating from heterologous organisms.

Said adjacent regulatory sequences are specific sequences such as promoters, enhancers, secretion signal sequences and/or terminators.

Another aspect of the invention is related to a vector comprising a nucleic acid molecule of the invention, possibly operably linked to one or more adjacent regulatory sequence(s) originating from homologous or from heterologous organisms.

In the present context "vector" is defined as any biochemical construct which may be used for the introduction of a nucleotide sequence (by transduction, transfection, transformation, infection, conjugation, etc.) into a cell.

Advantageously, a vector according to the invention is selected from the group consisting of plasmids (including replicative and integrative plasmids), viruses, phagemids, chromosomes, transposons, liposomes, cationic vesicles, or a mixture thereof. Said vector may already comprise one or more adjacent regulatory sequence(s), allowing the expression of said nucleic acid molecule and its transcription into a polypeptide of the invention.

The invention also relates to a FAD2 polypeptide having an amino acid substitution at (or corresponding to) position 216 relative to a wild type FAD2 protein.

Preferably, a FAD2 polypeptide of the invention comprises (or consists of) the amino acid sequence of SEQ ID NO 11 further comprising a substitution of serine for proline at position 216.

More preferably, a FAD2 polypeptide of the invention comprises (or consists of) the amino acid sequence of SEQ ID NO 15 further comprising a substitution of serine for proline at position 216.

A preferred FAD2 polypeptide of the invention comprises (or consists of) the amino acid sequence of SEQ ID NO 4.

The present invention also encompasses any fragments of a FAD2 protein of the invention having a delta-12 oleate desaturase activity and comprising said substitution or deletion at position 216.

Nucleic acid molecules, recombinant nucleic acid molecules, and/or vectors of the present invention are useful to transform target plants, and thereby confer altered FAD2 gene product, whereby the level of oleic acid is modified, preferably increased, in plant expressing a mutant FAD2 of the invention, compared to the corresponding level in a plant expressing the wild-type sequence.

The present invention is also related to a transformed host cell, or recombinant host cell, containing (or having incorporated) one or more of the nucleotide sequences and/or vectors according to the invention.

In the present context, a "transformed host cell" or "recombinant cell", also referred to as "transformant", is a cell having incorporated one or more of the nucleotide sequences and/or vectors according to the invention. The transformed host cell may be a cell in which said vector(s) and/or said nucleotide sequence(s) is/are introduced by means of genetic transformation, preferably by means of homologous recombination, or by any other well known methods used for obtaining a recombinant organism.

Any method by which the novel sequence can be incorporated into the host genome is contemplated by the present invention.

More particularly, any method by which the novel sequence can be incorporated into the host genome, and stably inherited by its progeny, is contemplated by the present invention.

A broad range of known techniques currently exist for achieving direct or indirect transformation of higher plants with exogenous nucleic acid molecules (e.g. exogenous DNA).

Transformation of plant cells can be mediated by the use of vectors. A common method of achieving transformation is the use of *Agrobacterium tumefaciens* to introduce a foreign gene into the target plant cell.

Plant viruses also provide a possible means for transfer of exogenous nucleic acid molecules (e.g. exogenous DNA).

Direct uptake of plant cells can also be employed. Typically, protoplasts of the target plant are placed in culture in the presence of the nucleic acid molecules to be transferred, and an agent which promotes the uptake of said nucleic acid molecules by protoplast. Useful agents in this regard are polyethylene glycol or calcium phosphate.

Alternatively, nucleic acid molecules uptake can be stimulated by electroporation. In this method, an electrical pulse is used to open temporary pores in a protoplast cell membrane, and said nucleic acid molecules in the surrounding solution are then drawn into the cell through the pores. Similarly, microinjection can be employed to deliver said nucleic acid molecules directly into a cell, and preferably directly into the nucleus of the cell.

In these techniques, transformation occurs in a plant cell in culture. Subsequent to the transformation event, plant cells can be regenerated to whole plants.

Techniques for the regeneration of mature plants from callus or protoplast culture are well known.

Alternate methods are also available which do not necessarily require the use of isolated cells, and therefore, plant regeneration techniques, to achieve transformation. These are generally referred to as "ballistic" or "particle acceleration" methods, in which nucleic acid molecules coated metal particles are propelled into plant cells by either a gunpowder charge or electrical discharge. In this manner, plant cells in culture or plant reproductive organs or cells, e.g. pollen, can be stably transformed with the nucleic acid molecules of interest.

The present invention can be applied to transformation of virtually any type of plant, monocotyledons or dicotyledons.

Suitable plants to be transformed are preferably oil producing crops, such as sunflower, soybean, cotton, corn, etc., preferably Brassica species, more preferably *Brassica napus* varieties.

In one aspect of the invention, a plant comprises at least one FAD2 coding sequence of the invention.

A plant of the invention can comprise a nucleic acid sequence of SEQ ID NO 1, 2 and/or 3, such as SPE04300-75.

SPE04300-75 variety is maintained as a Budapest Treaty patent deposit with NCIMB under accession number NCIMB 41445 made November 17, 2006.

Further examples of plants of the present invention comprising said SNP1884 mutation are OSE270, OSPE487 and Py1616 varieties.

OSE270 is maintained as a Budapest Treaty patent deposit with NCIMB under accession number 41407 made on June 14, 2006.

OSPE487 is maintained as a Budapest Treaty patent deposit with NCIMB under accession number NCIMB 41408 made on June 14, 2006.

PyL 616 is maintained as a Budapest Treaty patent deposit with NCIMB under accession number NCIMB 41406 made on June 14, 2006.

Another object of the invention is a method of producing high oleic plant lines comprising: (a) crossing a first plant with a second plant having at least one mutant FAD2 gene according to the invention, (b) obtaining seeds from the cross of step(a), (c) growing fertile plants from such seeds; (d) obtaining progeny seeds from the plants of step(c), and (e) identifying those seeds among the progeny that have high oleic acid content.

In another aspect, the invention provides a method for increasing the oleic acid content of plants, more particularly of Brassica plants, and preferably of *Brassica napus* plants comprising the steps of:
(a) inducing mutagenesis in at least some cells from a plant, more particularly of a Brassica plant, and preferably of a *Brassica napus* plant that has a oleic acid content of less than 70%;
(b) regenerating plants from at least one of said mutagenized cells;
(c) identifying and selecting regenerated plants which have a nucleic acid sequence of the invention and/or which expresses a FAD2 protein of the invention; and
(d) deriving further generations of plants from said regenerated plants.

Preferably, the seeds obtained from said plants provide an oil having an oleic acid content of more than 80wt.%, 81wt.%, 82wt.%, 83wt.%, or 84wt.%, more preferably of more than 85wt.%, 86wt.%, or even more preferably of more than 87wt.%, based upon the total weight of fatty acid present the oil.

Another object of the invention is a vegetable oil obtained from at least one plant according to the invention, which vegetable oil comprises more than (about) 80%, 81%, 82%, 83%, 84%, 85%, 86%, or 87% of oleic acid.

More particularly, a vegetable oil of the invention, obtained preferably from at least one Brassica species of the invention, more preferably from at least one *Brassica napus* variety according to the invention, comprises more than (about) 80%, 81%, 82%, 83%, 84%, 85%, 86%, or 87% of oleic acid. Said oil can further comprise less than (about) 4%, 3,5%, 3%, 2%, 1% or 0,5% of linolenic acid, based upon the total weight of the fatty acids present in the oil.

Preferably, said oil comprises more than (about) 80%, 81%, 82%, 83%, 84%, 85%, 86%, 87%, preferably between (about) 80% and (about) 88%, more preferably between (about) 80% and (about) 87,5% of oleic acid. Said oil can further comprise less than (about) 4%, 3,5%, 3%, 2%, 1%, or 0,5%, preferably between (about) 4% and (about) 0,4% of linolenic acid, based upon the total weight of the fatty acids present in the oil.

After an Ethyl Methane Sulfonate (EMS) treatment, a collection of high oleic winter oilseed rape varieties was grown during successive generations in the field, maintained and fixed by self-pollinations.

Their progenies were analysed for fatty acid composition using gas chromatography based analytical method, as commonly known in this area of technology.

Fatty acid composition was monitored in each generation and only material with oleic acid content higher than 75% was selected.

SPE04300-75, OSE270, OSPE487 and PyL 616 varieties exhibiting a very high oleic acid content have been obtained.

The line SPE04300-75 was thus sequenced.

Basic seed was used for the determination of fatty acid content in trials - small research trials (6 to 12 m²) or development trials (500 m²) and for the sequencing work.

### EXAMPLES

### Example 1

The seeds were grinded in a first solution consisting of methanol (800ml), trimethyl-pentane (200ml) and 5g of Na OH. About 3 ml of solution was used for about 10g of seeds (in other words about 10 to 50 seeds for 1 ml of solution) .

Extraction was performed during 20 minutes and thereafter a second solution, consisting of trimethylamine (900ml), and propanol, 2- (100ml), was added at the same volume as the first solution.

The resulting solution was vortexed and allowed to rest until formation of an upper phase.

The upper phase was sampled and transferred into viols.

One microliter of same was injected in a gas chromatograph (Fisons from thermo-electron with a columm DB3 -30 meter with a diameter of 0.25 mm and a thickness of 25 micrometer). Running time was about 4 min.

The oleic acid content results are summarized in table 1.

**Table 1.**

| Varieties | Oleic acid content (wt.%) | Appreciation | Linolenic acid content (wt.%) |
|---|---|---|---|
| PyL 616 | 80.5 - 85.1 | Very high | 2,3 - 2,6 |
| OSE270 | 86,4 - 87,5 | Very high | 3,5 - 3,8 |
| OSPE487 | 84,1 - 85,2 | Very high | 4,1 - 4,4 |
| SPE04300-75 | 84,4 - 85,6 | Very high | 4,7 - 5,0 |

In this example, from the seeds obtained in 2005 and 2006, the oleic acid content is higher than 80%, even higher than 84% in average, and is up to 87,5% based on the total weight of the fatty acid in the extracted oil. Besides, some varieties exhibit a low linolenic acid content, in particular a linolenic acid content equal or below 3.5%.

### Example 2

Plant materials used for sequencing are:
- mutant line with higher (very high) oleic fatty acid content (compared to wild type varieties) : SPE04300-75; and
- wild type varieties with normal oleic acid content: Bristol, Capitol, Vivol, Capvert and Caiman.

All these lines were grown in a growth chamber and the cotyledons and stems were collected from 7-day-old plants.

The plant tissues were freeze-dried and used for DNA extraction.

DNA was isolated with Qiagen Plant DNA kits (Qiagen INC.-USA, Valencia CA).

PCR was performed with TaqGold protocol (AB Biosystem, Inc,).

Reaction mix includes 2.5 µl 10x buffer, 0.2 µl TaqGold, 0.2 µl dNTP(25mM), 2 µl primers (5uM) and 10 ul DNA template (2ng/ul) and 10.1 ul N₂O.

PCR cycles were as follows: 94°C 5 min; 8 cycles of 94°C 40sec, 62°C 40sec, 72°C 1min, 94°°C 40sec, 60°C 40sec, 72°C 1min, 94°C 40sec, 58°C 40sec, 72°C 1 min, 94°C 40 sec, 56°C 40sec, 72°C 1 min; 3 cycles of 94°C 40sec, 55°C 40sec, 72°C 1 min; hold at 72°C for 7 min.

PCR products were analyzed on 1% agarose gel.

For sequencing, 5 µl PCR products were removed to a new tube and 1 µl ExonucleaseI (1:50 dilution) and 1 µl Shrimp Alkaline Phosphatase (1:5 dilution).

The mix was incubated at 37°C for 20 min and then 80°C for 15 min to inactivate the enzymes.

40 µl H₂O was added and 6 µl were used as template with 1 µl sequencing primer.

Sequencing was done on 3730 DNA Analyzer (Applied Biosystems).

Sequences were assembled and aligned using SeqMan II program of the LaserGene (DNASTAR, INC, Madison. WI).

### Example 3

Four *Brassica napus* delta-12 oleate desaturase (FAD2) gene sequences, 4684997, 46399190, 8705228 and 4092878, were downloaded from Genebank (NCBI). These sequences were used as queries to blast against Monsanto sequence database.

Using the "blastn" programs (NCBI), a number of high score hits were obtained. All the hit sequences were downloaded and reassembled with the SeqmanII program (DNASTAR Inc, Madison, Wisconsin, USA).

Two distinct transcripts were identified and designated as Fad2-1 (SEQ ID NO 9) and Fad2-2 (SEQ ID NO 13). Fad2-1 and Fad2-2 share a high sequence homology, with 97 % sequence identity.

Having regard to SPE04300-75 variety, there is 96% sequence identity between the two transcripts Fad2-1 (SEQ ID NO 5) and Fad2-2 (SEQ ID NO 2).

To identify causative mutations associated with high oleic acid content in the mutant lines and their progenies, nested locus-specific primers were designed to cover the entire sequences.

The 3' end of a primer was always located at a nucleotide that differentiated Fad2-1 from Fad2-2 except those located at 5' and 3' ends of the consensus sequences where there was not differential nucleotide between the two genes.

The primers were also designed in such way that one amplicon would overlap with another to ensure full coverage of the entire sequence. These primers were arrayed and used to generate locus-specific amplicons on mutants and wild types. Sequencing results indicated that all the locus-specific PCR primers behaved as expected.

Sequences belonging to the same gene were assembled together using SeqManII program.

The consensus genomic sequences of the wild type Fad2-1 and Fad2-2 genes are represented respectively by SEQ ID NO 8 and 12.

Table 2 summarizes the sequence features of both Fad2-1 and Fad2-2 genes.

**Table 2:**

| Features | FAD2-1 position | FAD2-2 position |
|---|---|---|
| Gene | 1 - 2614 | 1 - 2666 |
| 5' UTR | 1 - 1218 | 1 - 1238 |
| Exon | 1 - 108 | 1 - 111 |
| Intron | 109 - 1213 | 112 - 1234 |
| Exon | 1214 - 2614 | 1235 - 2619 |
| CDS | 1218 - 2372 | 1239 - 2393 |
| 3'UTR | 2373 - 2614 | 2394 - 2666 |

The features are based on the consensus genomic sequences from multiple reads on different genotypes.

Both Fad2-1 and Fad2-2 genes have one intron each.

The intron sizes are slightly different between the two genes. For Fad2-1, intron spans 1105 base pairs starting from position 109 to 1213, while for Fad2-2, intron consists of 1123 base pairs starting from position 112 to 1234 on the consensus sequences.

The intron is located at 5'UTR region.

Putative translation initiation codons are located at 1218 and 1239 for Fad2-1 and Fad2-2 genes, respectively.

The translation termination codons are located at 2370-2372 and 2391-2393, respectively for Fad2-1 and Fad2-2.

3'UTR sequences are 242 base pairs for Fad2-1 and 273 base pairs for Fad2-2 genes.

A point mutation was found at position 1884 (called SNP1884) of FAD2-2 gene (as represented by SEQ ID NO 1), which caused a change in genetic codon from CCC to TCC, resulting in an alternation of amino acid residue from proline to serine.

It appears that said proline at position 216 is a conformationally important amino acid and its replacement with serine is responsible for a radical change in the enzyme function in the mutant line.

## Claims

1. An isolated nucleic acid molecule comprising a nucleic acid sequence encoding a delta-12 oleate desaturase (FAD2) protein, said FAD2 protein having a substitution for proline at position 216 relative to a wild-type FAD2 protein.

2. An isolated nucleic acid molecule according to claim 1, wherein said FAD2 protein is a *Brassica* FAD2 protein, more particularly a *Brassica napus* FAD2 protein.

3. An isolated nucleic acid molecule according to claim 1 or 2, wherein said FAD2 protein has a substitution of non-ionisable polar amino acid for proline.

4. An isolated nucleic acid molecule according to any of claims 1 to 3, wherein said non-ionisable polar amino acid is serine.

5. An isolated nucleic acid molecule according to any of claims 1 to 4 comprising a nucleic acid of SEQ ID NO 1, 2, or 3, its complementary form or RNA form.

6. An isolated nucleic acid molecule comprising a nucleotide sequence having at least 80%, preferably at least 85%, more preferably at least 90% and even more preferably at least 95%, 96%, 97%, 98% or 99% identity with SEQ ID NO 1, 2 or 3, or with the complementary form or RNA form thereof, encoding a FAD2 protein having an amino acid substitution at position 216 relative to a wild-type FAD2 protein.

7. An isolated nucleic acid molecule according to any of claims 1 to 9, wherein said wild-type FAD2 protein comprises an amino acid sequence of SEQ ID NO 11 or 15.

8. A fragment of at least 10 nucleotides of an isolated nucleic acid molecule according to any of claims 1 to 7, said fragment comprising the mutated codon corresponding to said amino acid substitution at position 216.

9. A FAD2 protein having an amino acid substitution at position 216, or corresponding to position 216, relative to a wild-type FAD2 protein such as the wild-type FAD2 protein represented by the amino acid sequence of SEQ ID NO 11 or 15.

10. A FAD2 protein according to claim 9, comprising an amino acid sequence of SEQ ID NO 4.

11. A vector comprising a nucleic acid molecule according to any of claims 1 to 7.

12. A host cell comprising a nucleic acid sequence according to any of claims 1 to 7 or a vector according to claim 11.

13. A plant stably transformed with a vector of claim 11.

14. A plant of claim 13, wherein said plant to be transformed is selected from the group consisting of oil producing crops.

15. A plant of claim 14, wherein said oil producing crops are sunflowers, soybeans, cottons, corns and/or rapeseeds.

16. A plant or a plant part or a seed containing a nucleic acid molecule according to any of claims 1 to 7, or a protein according to claim 9 or 10.

17. A plant or a plant part or a seed according to claim 16 obtainable by a mutagenesis treatment, more particularly by an EMS treatment.

18. A seed derived from a plant according to any of claims 13 to 17.

19. Progeny derived from a plant or a plant part or a seed according to any of claims 13 to 18.

20. A vegetable oil obtained from seeds according to claim 18, comprising more than (about) 80% or more than (about) 84%, preferably more than (about) 85% of oleic acid based upon the total weight of the fatty acids present in the oil.

21. A vegetable oil according to claim 20 further comprising less than (about) 4%, 3,5%, 3%, 2%, 1% or 0,5% of linolenic acid.

22. A food or a feed product containing and/or prepared with a vegetable oil according to claim 20 or 21.

23. A food or a feed product containing and/or prepared with a plant, a plant part or a seed according to any of claims 13 to 19.

24. A vegetable meal containing and/or prepared with a plant, a plant part or a seed according to any of claims 13 to 19.

25. A method of enhancing the oleic acid content in a plant comprising transforming a plant with the vector of claim 11.

26. Use of a fragment of at least 10 nucleotides according to claim 8 as a primer, probe and/or selectable marker.

27. A method of marker assisted selection of plants of *Brassica* species using a nucleic acid molecule of any of claims 1 to 8.

28. An assay kit comprising a first container containing a nucleic acid molecule of any of claims 1 to 8.

29. A method of producing high oleic plant lines comprising:
(a) crossing a first plant according to any of claims 13 to 19 with a second plant,
(b) obtaining seeds from the cross of step (a),
(c) growing fertile plants from such seeds,
(d) obtaining progeny seeds from the plants of step(c), and
(e) identifying those seeds among the progeny that have high oleic acid content.

30. A method of producing high oleic plant lines comprising:
(a) inducing mutagenesis in at least some cells from a plant, more particularly of a Brassica plant, and preferably of a *Brassica napus* plant that has a oleic acid content of less than 70%;
(b) regenerating plants from at least one of said mutagenized cells;
(c) identifying and selecting regenerated plants which have a nucleic acid sequence according to any of claims 1 to 8 and/or which expresses a FAD2 protein according to claim 9 or 10; and
(d) deriving further generations of plants from said regenerated plants.
